# EUROPEAN PATENT SPECIFICATION

(11) **EP 2 447 334 B1**
(45) Date of publication and mention of the grant of the patent: **17.09.2014**
(21) Application number: 10792365.8
(22) Date of filing: 25.06.2010
(51) Int. Cl.: C09K 11/06, H05B 33/10, H05B 33/14

(54) **COMPOUND FOR AN ORGANIC PHOTOELECTRIC ELEMENT, AND AN ORGANIC PHOTOELECTRIC ELEMENT COMPRISING THE SAME**
VERBINDUNG FÜR EIN ORGANISCHES PHOTOELEKTRISCHES ELEMENT UND ORGANISCHES PHOTOELEKTRISCHES ELEMENT DAMIT
COMPOSÉ POUR ÉLÉMENT PHOTOÉLECTRIQUE ORGANIQUE, ET ÉLÉMENT PHOTOÉLECTRIQUE ORGANIQUE LE COMPRENANT

(30) Priority: 25.06.2009 KR 20090057234
(43) Date of publication of application: 02.05.2012
(73) Proprietor: CHEIL INDUSTRIES INC., Kumi-city Gyeongsangbuk-do 730-030 (KR)
(72) Inventor: JUNG, Sung-Hyun, Uiwang-si Gyeonggi-do 437-711 (KR); KIM, Young-Hoon, Uiwang-si Gyeonggi-do 437-711 (KR); KIM, Hyung-Sun, Uiwang-si Gyeonggi-do 437-711 (KR); LEE, Ho-Jae, Uiwang-si Gyeonggi-do 437-711 (KR); YU, Eun-Sun, Uiwang-si Gyeonggi-do 437-711 (KR); CHAE, Mi-Young, Uiwang-si Gyeonggi-do 437-711 (KR)
(74) Representative: Michalski Hüttermann & Partner Patentanwälte mbB
(86) International application number: PCT/KR2010/004156
(87) International publication number: WO 2010/151083

(56) References cited:
- EP-A1- 1 777 227
- DE-A1-102006 031 990
- KR-A- 20060 025 933
- KR-A- 20100 003 624
- US-A- 5 942 340
- US-A- 5 952 115
- US-A1- 2006 063 033
- US-A1- 2008 124 455
- US-A1- 2008 124 455

## Description

### [Technical Field]

This disclosure relates to a compound for an organic photoelectric device and an organic photoelectric device including the same.

### [Background Art]

An organic photoelectric device is, in a broad sense, a device for transforming photo-energy to electrical energy or a device for transforming electrical energy to photo-energy conversely. As examples, the organic photoelectric device includes an organic light emitting diode (OLED), a solar cell, a transistor, and the like. Particularly, an organic light emitting diode has recently drawn attention due to the increase in demand for flat panel displays.

When current is applied to an organic light emitting diode, holes are injected from an anode and electrons are injected from a cathode, then injected holes and electrons move to each hole transport layer (HTL) and electron transport layer (ETL) and recombined to a light emitting exciton in an emission layer. The light emitting excitons generate lights while shifting to a ground state. The light emission material may be classified as a fluorescent material including singlet excitons and a phosphorescent material including triplet excitons according to light emitting mechanism. The fluorescent and phosphorescent materials may be used for a light emitting source of an organic light emitting diode (D. F.O'Brien, Appl. Phys. Lett., 74 3, 442, 1999; M. A. Baldo, Appl. Phys. lett., 75 1, 4, 1999).

When electrons are transported from the ground state to the exited state, a single exciton undergoes non-light emitting transition to a triplet exciton through intersystem crossing and the triplet exciton is transited to the ground state to emit light. Herein, such a light emission refers to phosphorescent emission. When the triplet exciton is transited, it cannot directly transit to the ground state. Therefore, it is transited to the ground state after the electron spin is flipped

Therefore, a half-life (light emitting time, lifetime) of phosphorescent emission is longer than that of fluorescent emission.

When holes and electrons are recombined to produce a light emitting exciton, three times triplet light emitting excitons are produced compared to the amount of the singlet light emitting excitons. A fluorescent material has 25% of the singlet-exited state and a limit in luminous efficiency. On the other hand, a phosphorescent material can utilize 75% of the triplet exited state and 25% of the singlet exited state, so it can theoretically reach 100% of the internal quantum efficiency. Accordingly, the phosphorescent light emitting material has advantages of accomplishing around four times more luminous efficiency than the fluorescent light emitting material.

Meanwhile, a dopant along with the host material may be included in an emission layer to increase efficiency and stability of organic light emitting diode. For the host material, 4-N,N-dicarbazolebiphenyl (CBP) has been mainly used. However, CBP has high structural symmetry and may be easily crystallized. Due to low thermal stability, short-circuit or a pixel defect may occur during heat resistance test of a device. Furthermore, since host materials such as CBP has faster hole transport speed than electron transport speed, an exciton may not be effectively formed in an emission layer, decreasing luminous efficiency of a device.

In addition, a low molecular host material is in general using a vacuum-deposition, the vacuum deposition may cost more than a wet process. In addition, most of low molecular host materials have low solubility against an organic solvent, they may not be applied in a wet process and thus, form an organic thin layer having excellent film characteristics.

Accordingly, in order to realize an organic photoelectric device with excellent efficiency and life-span, there are needs for development of a phosphorescent host material and a charge transport material having excellent electrical and thermal stability and bipolar characteristics well-transporting both holes and electrons or a host material mixed with a material being capable of transporting holes and electrons well.

Compounds for organic photoelectronic devices are inter alia disclosed in the US 20081124455, US 2006/063033 and EP 1777227.

### [DISCLOSURE]

### [Technical Problem]

One embodiment of the present invention provides a compound for an organic photoelectric device having excellent thermal stability, and being capable of transporting holes and electrons well.

Another embodiment of the present invention provides an organic photoelectric device having excellent efficiency and a driving voltage by including the compound for an organic photoelectric device.

Yet another embodiment of the present invention provides a display device including the organic photoelectric device.

### [Technical Solution]

According to one embodiment of the present invention, a compound for an organic photoelectric device in which substituents represented by the following Chemical Formulas 1 to 3 are sequentially combined is provided.

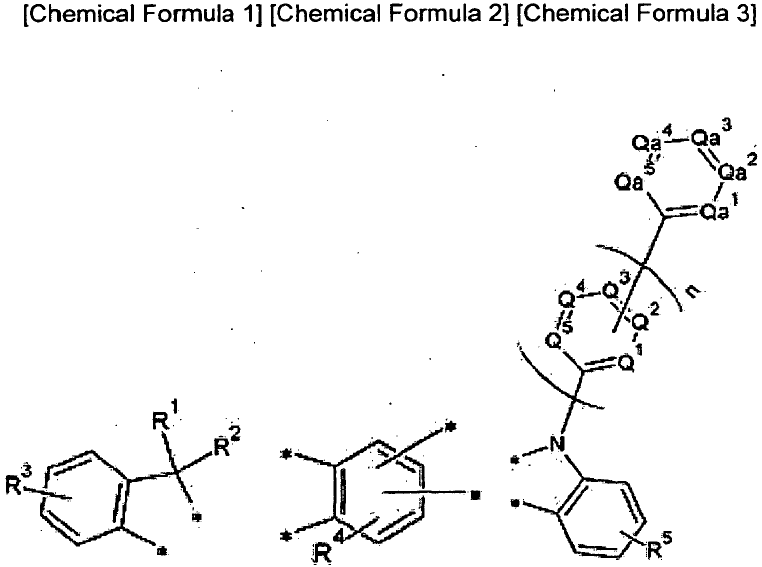

In Chemical Formulas 1 to 3, '

Q₁ to Q⁵ and Qa¹ to Qa⁵ are the same or different and are each independently N or CR, wherein R is hydrogen, a substituted or unsubstituted C6 to C30 aryl group, a substituted or unsubstituted C3 to C30 heteroaryl group, or a combination thereof,
R¹ to R⁵ are the same or different and are each independently hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof, and
n is an integer ranging from 0 to 5.

The Chemical Formula 1 may be represented by the following Chemical Formula 1a.

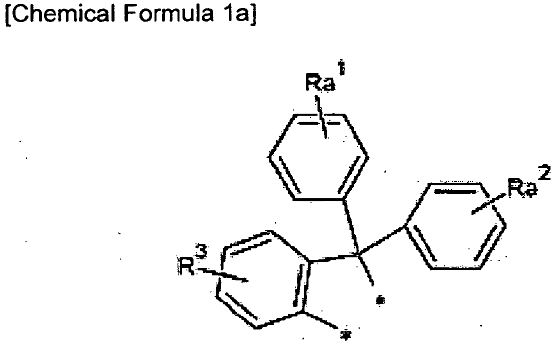

In Chemical Formula 1a,

Ra¹ and Ra² are the same or different and are each independently hydrogen, a substituted or unsubstituted carbazolyl group, a substituted or unsubstituted C6 to C30 aryl group, a substituted or unsubstituted C6 to C30 arylamine group, or a combination thereof,

R³ is hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof.

The above Chemical Formula 2 may be represented by the following Chemical Formula 2a or 2b.

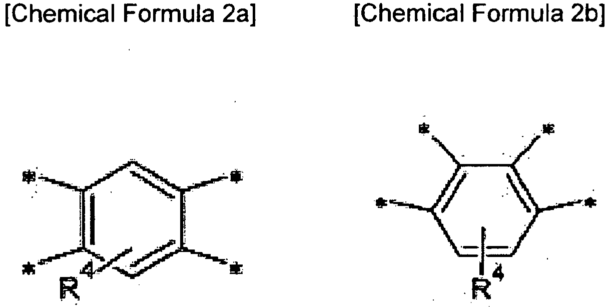

In Chemical Formulas 2a and 2b,

R⁴ is hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof.

In addition, Q¹ to Q⁵ and Qa¹ to Qa⁵ in the above Chemical Formula 3 are the same or different and each independently N or CR, provided that at least one selected from Q¹ to Q⁵ and Qa¹ to Qa⁵ is N, and the rest of them are each independently CR, wherein the R may be hydrogen, a substituted or unsubstituted C6 to C30 aryl group, a substituted or unsubstituted C3 to C30 heteroaryl group, or a combination thereof and particularly, one to three selected from Q¹ to Q⁵ are N, one to three selected from Qa¹ to Qa⁵ is N, and the rest of them are each independently CR, wherein the R may be hydrogen, a substituted or unsubstituted C6 to C30 aryl group, a substituted or unsubstituted C3 to C30 heteroaryl group, or a combination thereof. In addition, n in the above Chemical Formula 3 may be an integer ranging from 0 to 2.

The Chemical Formula 3 may be represented by the following Chemical Formula 3a.

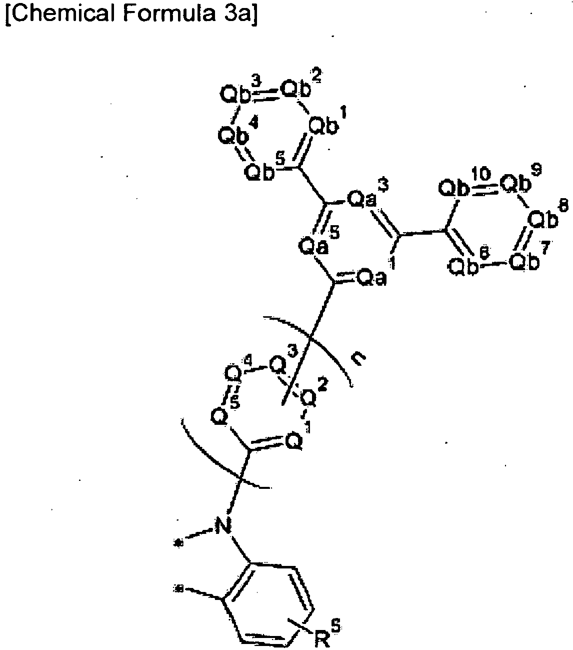

In Chemical Formula 3a,
Qa¹, Qa³ and Qa⁵ are the same or different, and are each independently N or CH, provided that one or more of Qa¹, Qa³ and Qa⁵ is N,
Q¹ to Q⁵ and Qb¹ to Qb¹⁰ are the same or different and are each independently N or CR, wherein R is hydrogen, a substituted or unsubstituted C6 to C30 aryl group, a substituted or unsubstituted C3 to C30 heteroaryl group, or a combination thereof,
R⁵ is hydrogen, a carbazolyl group, a substituted or unsubstituted C6 to C30 aryl group, a substituted or unsubstituted C3 to C30 heteroaryl group, a substituted or unsubstituted C6 to C30 arylamine group, or a combination thereof, and
n is an integer ranging from 0 to 5.

In Chemical Formulas 1 to 3, R³ to R⁵ are the same or different and are each independently hydrogen, a C1 to C30 alkyl group, a C6 to C30 aryl group, or a combination thereof.

The compound for an organic photoelectric device may be represented by the following Chemical Formulas 4 to 9.

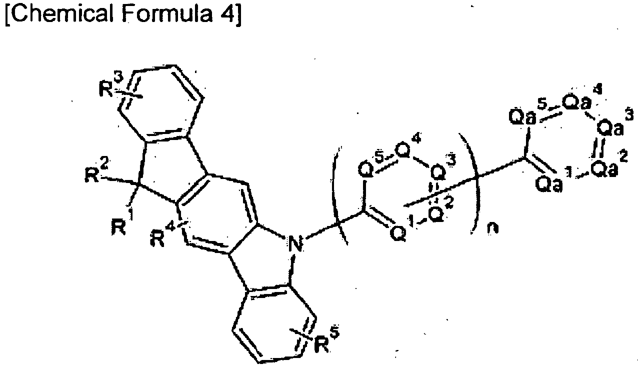

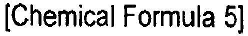

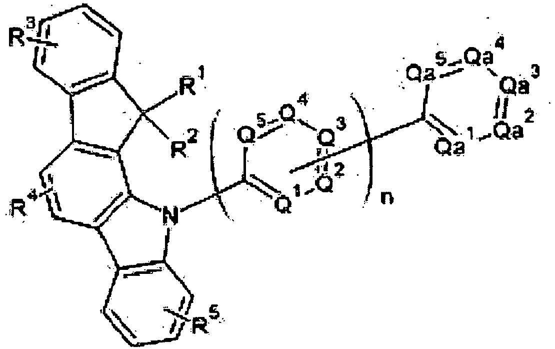

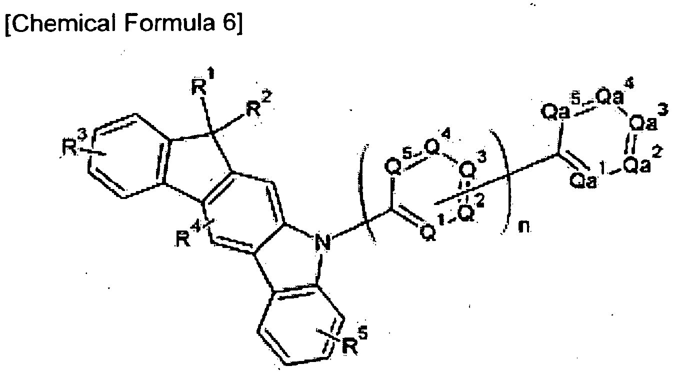

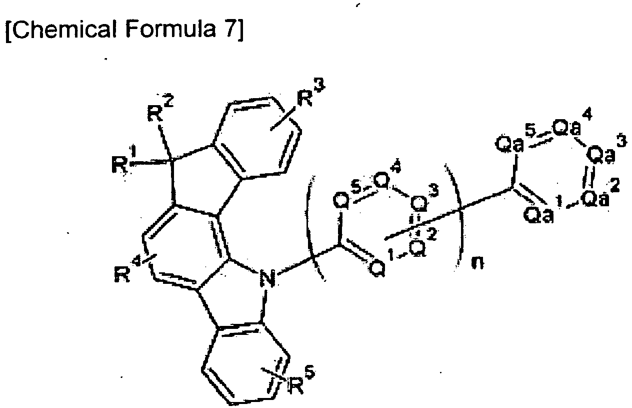

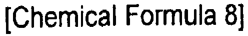

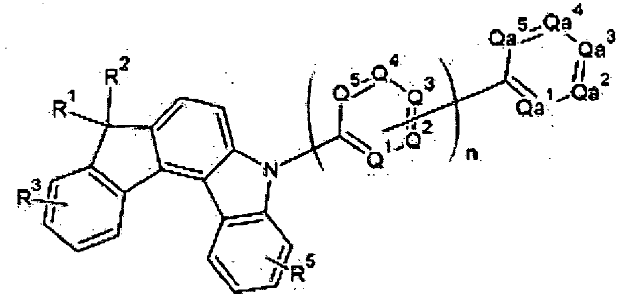

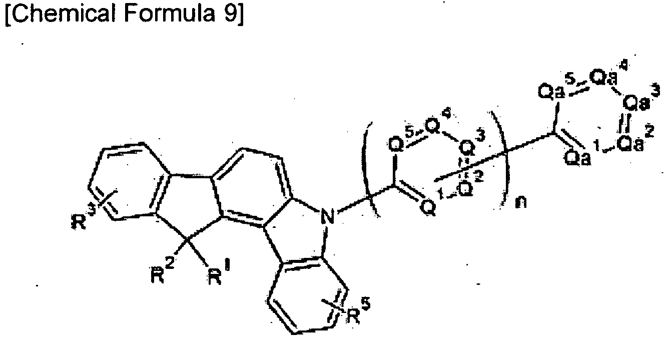

In Chemical Formulas 4 to 9,
Q¹ to Q⁵ and Qa¹ to Qa⁵ are the same or different and are each independently N or CR, wherein R is hydrogen, a substituted or unsubstituted C6 to C30 aryl group, a substituted or unsubstituted C3 to C30 heteroaryl group, or a combination thereof,
R¹ to R⁵ are the same or different and are each independently hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof, and
n is an integer ranging from 0 to 5.

Particularly, the compound for an organic photoelectric device may be one represented by the following Chemical Formulas 10 to 33.

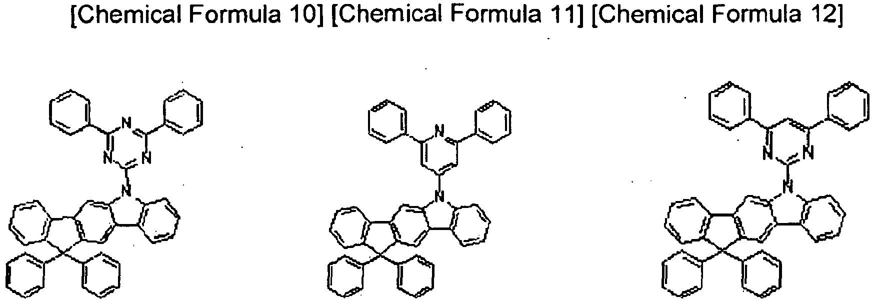

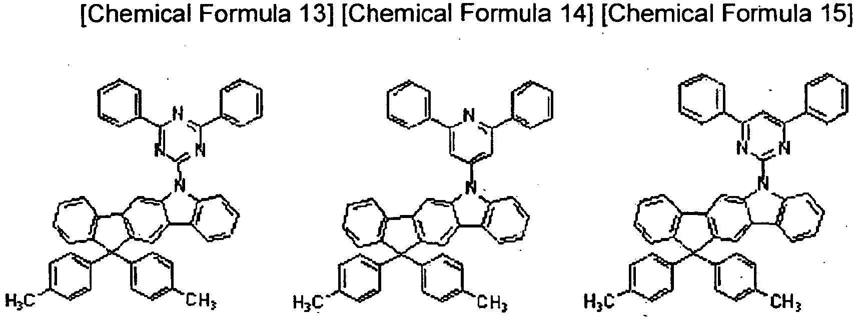

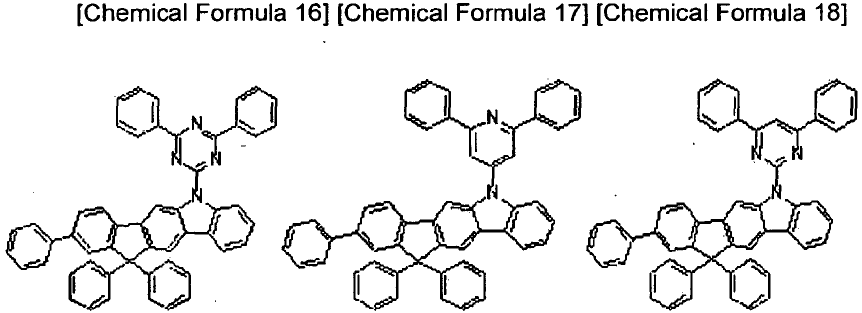

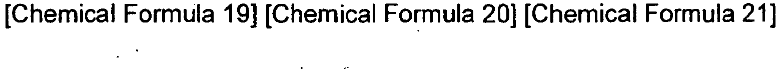

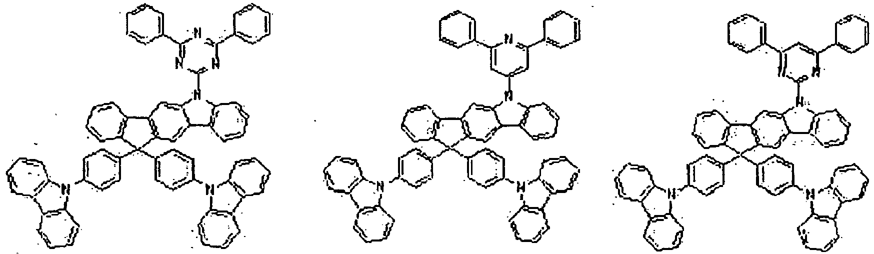

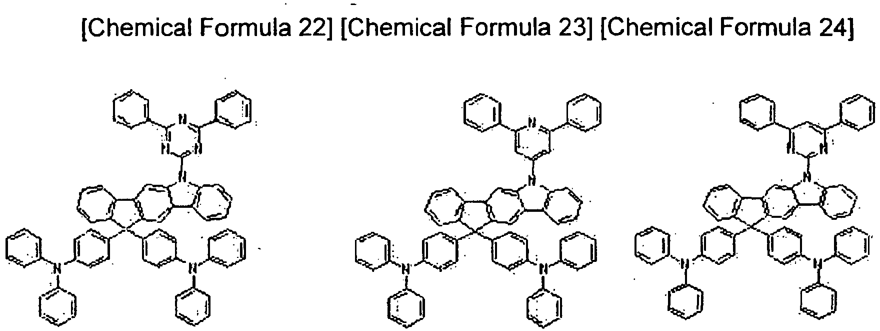

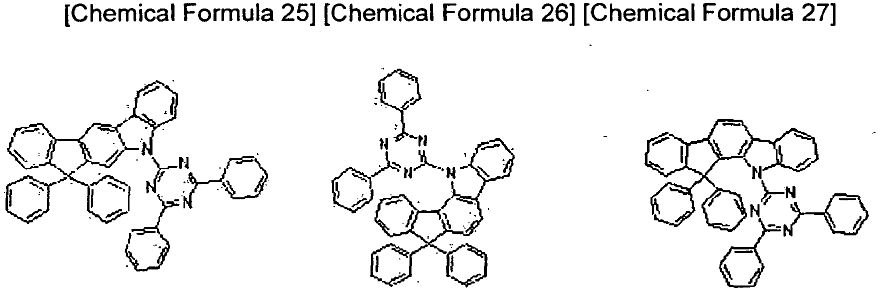

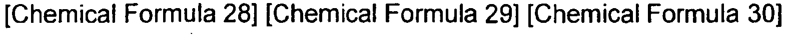

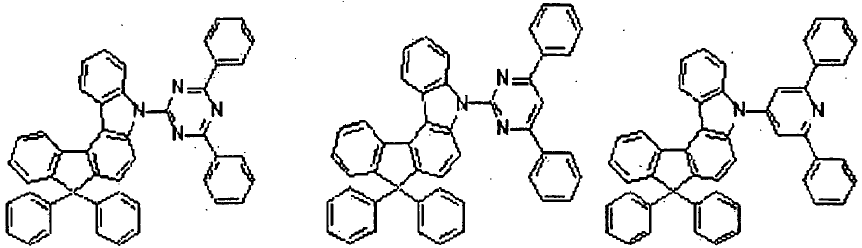

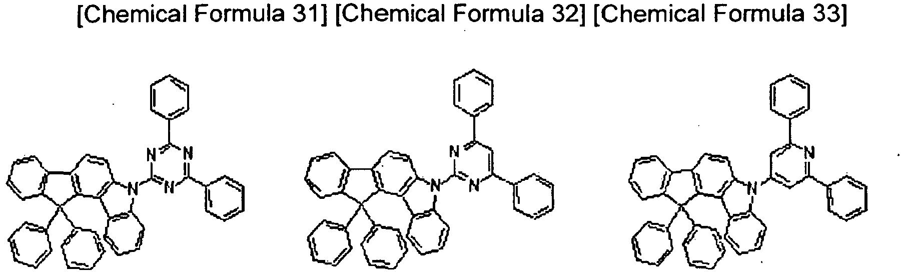

The compound for an organic photoelectric device may be used as a charge transport material or a host material, and has a thermal decomposition temperature (Td) of 350 °C to 600 °C.

According to another embodiment of the present invention, provided is an organic photoelectric device that includes an anode, a cathode and at least one or more organic thin layer between the anode and the cathode, wherein the organic thin layer includes the compound for an organic photoelectric device.

The organic thin layer may be an emission layer, a hole blocking layer, an electron blocking layer, an electron transport layer (ETL), an electron injection layer (EIL), a hole injection layer (HIL), a hole transport layer (HTL), or a combination thereof.

According to yet another embodiment of the present invention, a display device including the organic photoelectric device is provided.

Hereinafter, further embodiments of the present invention will be described in detail.

### [Advantageous Effects]

The compound for an organic photoelectric device according to one embodiment has excellent thermal stability, and particularly, may be applied to an organic thin layer of an organic photoelectric device and thus can provide an organic photoelectric device and a display device having high luminous efficiency at a low voltage and improved life-span.

### [Description of the Drawings]

FIGS. 1 to 5 are cross-sectional views showing organic photoelectric devices including compounds according to various embodiments of the present invention.
FIG. 6 is a graph showing current density change depending on voltage of the organic photoelectric devices according to Example 3 and Comparative Example 1.
FIG. 7 is a graph showing luminance change depending on voltage of the organic photoelectric devices according to Example 3 and Comparative Example 1.
FIG. 8 is a graph showing current efficiency change depending on luminance of the organic photoelectric devices according to Example 3 and Comparative Example 1.
FIG. 9 is a graph showing electric power efficiency change depending on luminance of the organic photoelectric devices according to Example 3 and Comparative Example 1.

### <Description of Reference Numerals Indicating Primary Elements in the Drawings>

100 : organic photoelectric device 110 : cathode
120 : anode 105 : organic thin layer
130 : emission layer 140 : hole transport layer (HTL)
150 : electron transport layer (ETL) 160 : electron injection layer (EIL)
170 : hole injection layer (HIL) 230 : emission layer + electron transport layer (ETL)

### [Mode for Invention]

Exemplary embodiments of the present invention will hereinafter be described in detail. However, these embodiments are only exemplary, and the present invention is not limited thereto but rather is defined by the scope of the appended claims.

In the present specification, the term "substituted", when a definition is not otherwise provided, refers to one substituted with a halogen group, a cyano group, a C1 to C30 alkyl group, a C3 to C30 cycloalkyl group, a C6 to C30 aryl group, a C1 to C30 alkoxy group, or a combination thereof.

In the present specification the term "halogen group", when a definition is not otherwise provided, refers to a fluoro group, a chloro group, a bromo group, or a combination thereof, and particularly, a fluoro group.

In the present specification, the term "hetero", when a definition is not otherwise provided, refers to one including 1 to 3 of N, O, S, P, and remaining carbons in one ring.

According to one embodiment of the present invention, a compound for an organic photoelectric device in which substituents represented by the following Chemical Formulas 1 to 3 are sequentially combined is provided.

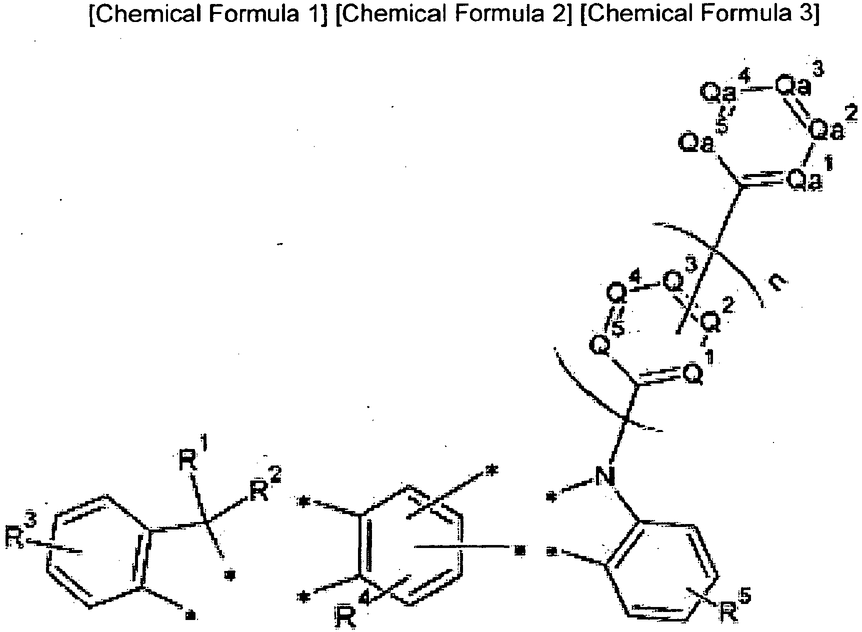

In Chemical Formulas 1 to 3,
Q¹ to Q⁵ and Qa¹ to Qa⁵ are the same or different and are each independently N or CR, wherein R is hydrogen, a substituted or unsubstituted C6 to C30 aryl group, a substituted or unsubstituted C3 to C30 heteroaryl group, or a combination thereof, and when each Q¹ to Q⁵ and Qa¹ to Qa⁵ is independently CR, each R is the same or different. In addition, when the R is substituted with a C1 to C30 alkyl group, the compound is applied to an organic thin layer of organic photoelectric device and may improve film formation characteristics of the organic thin layer.

When R is a substituted or unsubstituted C6 to C30 aryl group, the aryl group may be a phenyl group, a naphthyl group, an anthracene group, a phenanthrene group, a tetracene group, a pyrene group, a fluorine group, or a combination thereof. However, the aryl group is not limited to the above described.

When R is a substituted or unsubstituted C3 to C30 heteroaryl group, the heteroaryl group may be thiophene, furan, pyrrole, imidazole, thiazole, oxazole, oxadiazole, thiadiazole, triazole, triazine, pyridine, pyrimidine, pyridazine, pyrazine, quinoline, isoquinoline, or a combination thereof. However, the heteroaryl group is not limited to the above described.

R¹ to R⁵ are the same or different and are each independently hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof, n is an integer ranging from 0 to 5. When n is an integer of 2 or more, and each repeating unit may be the same or different.

The Chemical Formula 1 may be represented by the following Chemical Formula 1a.

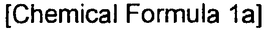

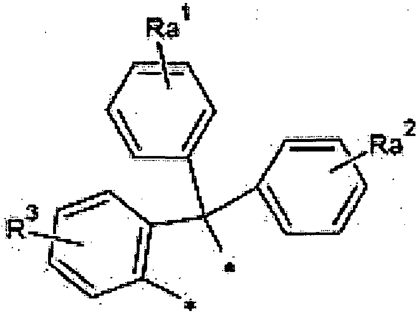

In Chemical Formula 1a,
Ra¹ and Ra² are the same or different and are each independently hydrogen, a substituted or unsubstituted carbazolyl group, a substituted or unsubstituted C6 to C30 aryl group, a substituted or unsubstituted C6 to C30 arylamine group, or a combination thereof,
R³ is hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof.

The Chemical Formula 2 may be represented by the following Chemical Formula 2a or 2b.

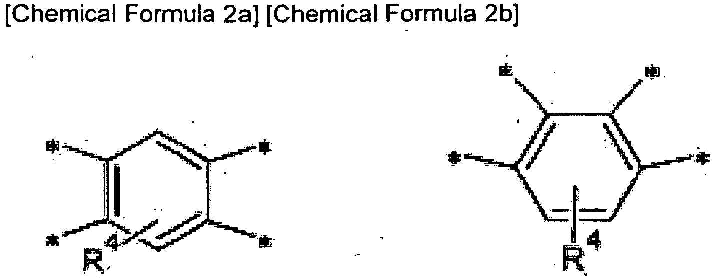

In Chemical Formulas 2a and 2b,
R⁴ is hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof.

In addition, Q¹ to Q⁵ and Qa¹ to Qa⁵ in the above Chemical Formula 3 are the same or different and each independently N or CR, provided that at least one selected from Q¹ to Q⁵ and Qa¹ to Qa⁵ is N, the rest of them are each independently CR, wherein the R may be hydrogen, a substituted or unsubstituted C6 to C30 aryl group, a substituted or unsubstituted C3 to C30 heteroaryl group, or a combination thereof. In particular, one to three selected from Q¹ to Q⁵ is N, one to three selected from Qa¹ to Qa⁵ is N, and the rest of them are each independently CR, wherein the R may be hydrogen, a substituted or unsubstituted C6 to C30 aryl group, a substituted or unsubstituted C3 to C30 heteroaryl group, or a combination thereof. Accordingly, the compound may include a substituent better functioning an electron transporting group.

Furthermore, n in the above Chemical Formula 3 may be an integer ranging from 0 to 2.

The Chemical Formula 3 may be represented by the following Chemical Formula 3a.

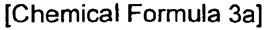

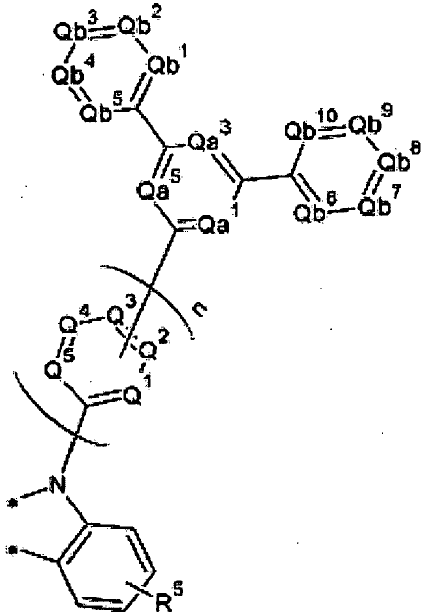

In Chemical Formula 3a,
Qa¹, Qa³ and Qa⁵ are the same or different, and are each independently N or CH, provided that one or more of Qa¹, Qa³ and Qa⁵ is N,
Q¹ to Q⁵ and Qb¹ to Qb¹⁰ are the same or different and are each independently N or CR, wherein R is hydrogen, a substituted or unsubstituted C6 to C30 aryl group, a substituted or unsubstituted C3 to C30 heteroaryl group, or a combination thereof,
R⁵ is hydrogen, a carbazolyl group, a substituted or unsubstituted C6 to C30 aryl group, a substituted or unsubstituted C3 to C30 heteroaryl group, a substituted or unsubstituted C6 to C30 arylamine group, or a combination thereof, and
n is an integer ranging from 0 to 5.

In Chemical Formulas 1 to 3, R³ to R⁵ are the same or different and are each independently hydrogen, a C1 to C30 alkyl group, a C6 to C30 aryl group, or a combination thereof.

The compound for an organic photoelectric device may be represented by the following Chemical Formulas 4 to 9.

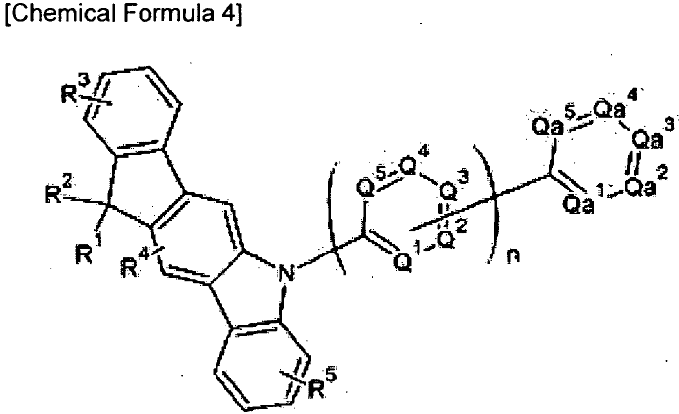

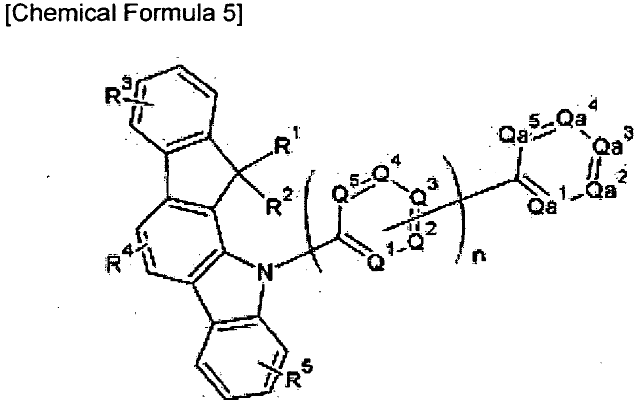

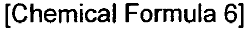

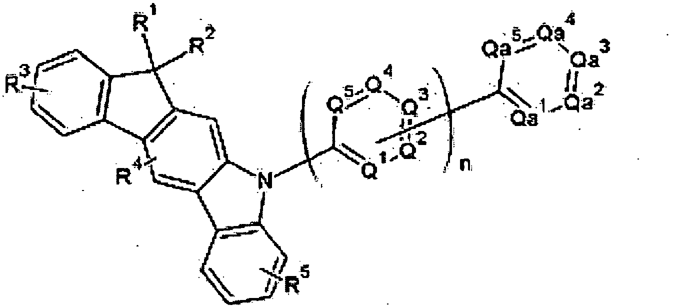

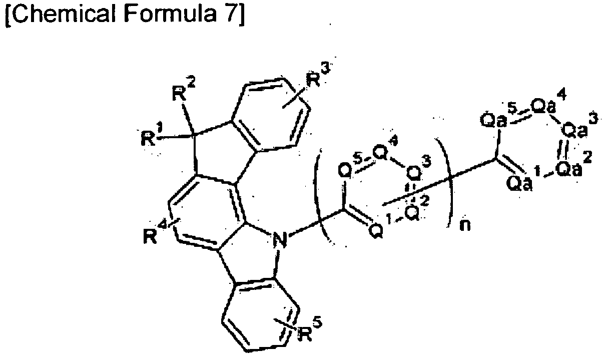

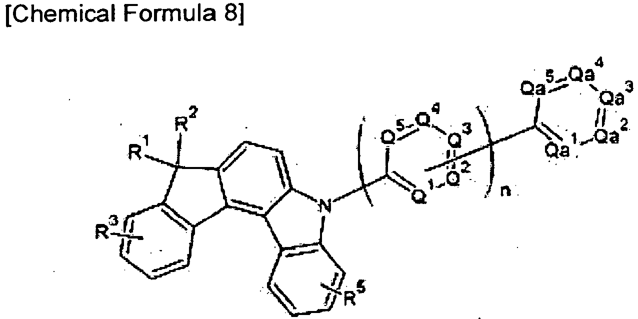

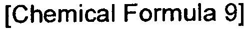

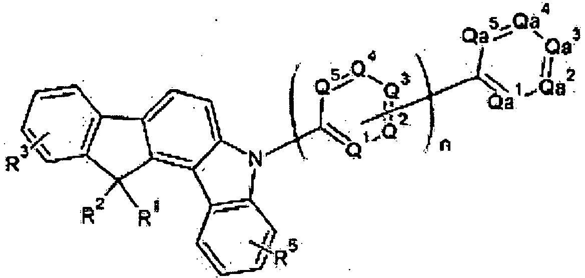

In Chemical Formulas 4 to 9,
Q¹ to Q⁵ and Qa¹ to Qa⁵ are the same or different and are each independently N or CR, wherein R is hydrogen, a substituted or unsubstituted C6 to C30 aryl group, a substituted or unsubstituted C3 to C30 heteroaryl group, or a combination thereof,
R¹ to R⁵ are the same or different and are each independently hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof, and
n is an integer ranging from 0 to 5.

Particularly, the compound for an organic photoelectric device may be those represented by the following Chemical Formulas 10 to 33. The compound for an organic photoelectric device according to one embodiment is not limited thereto.

The compound for an organic photoelectric device may be used as a charge transport material or a host material, and particularly, when the compound for an organic photoelectric device may be used as a host material, the compound is a phosphorescent host material that lowers a driving voltage of an organic photoelectric device, and improves luminous efficiency.

When the compound for an organic photoelectric device is a host material, the compound for an organic photoelectric device may be used as a mixture or blend with a generally-used low molecular host material or a polymer host material. In addition, a binder resin such as polyvinylcarbazole, polycarbonate, polyester, poly arylate, polystyrene, acryl polymer, methacryl polymer, polybutyral, polyvinylacetal, a diallylphthalate polymer, phenolic resin, an epoxy resin, a silicone resin, polysulfone resin, or a urea resin may be mixed.

For example, for the low molecular host material, the compounds represented by the following Chemical Formulas 34 to 37, and for the polymer host material, a polymer having a conjugated double bond such as a fluorene-based polymer, a polyphenylenevinylene-based polymer, a polyparaphenylene-based polymer, and the like may be used. However, the low molecular host material and polymer host material are not limited to the above described.

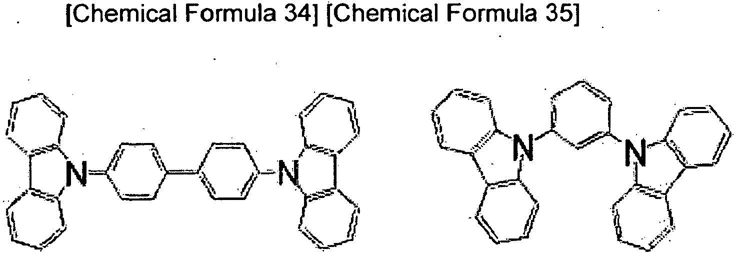

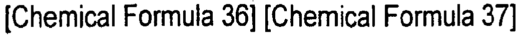

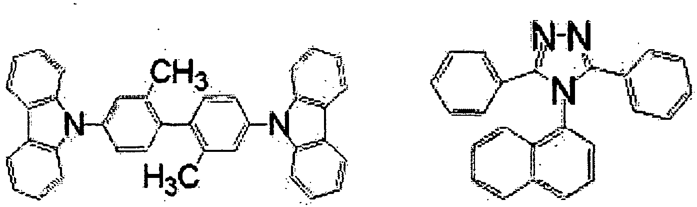

When the compound for an organic photoelectric device is used as a host material, the compound for an organic photoelectric device may be used singularly or along with a dopant as a host material. The dopant is a compound having a high emission property, by itself. However, it is usually added to a host in a minor amount, so it is also called a guest. The dopant may be a light-emitting material while being doped on a host material. Generally, a material such as a metal complex being capable of light-emitting by multiplet excitations such as triplet excitation or more is used for a dopant. Such a dopant may be a generally-used red (R), green (G), blue (B), or white (W) fluorescent or phosphorescent dopant, and particularly, red, green and blue, or white phosphorescent dopant is preferable. A material that has high luminous efficiency, is not agglomerated, and is distributed in a host material uniformly may be used.

The phosphorescent dopant may be an organic metal compound including an element that is lr, Pt, Os, Ti, Zr, Hf, Eu, Tb, Tm, Fe, Co, Ni, Ru, Rh, Pd, or a combination thereof. More particularly, a red phosphorescent dopant may include platinum-octaethylporphyrin complex (PtOEP), Ir(btp)₂(acac) (bis(2-(2'-benzothienyl)-pyridinato-N,C3')iridium(acetylacetonate)), Ir(Piq)₂(acac), Ir(Piq)₃, RD61 (UDC), and the like, a green phosphorescent dopant may include Ir(PPy)₂(acac), Ir(PPy)₃, GD48 (UDC), and the like, and a blue phosphorescent dopant may include (4,6-F₂PPy)₂Irpic, flrpic(Ir bis[4,6-di-fluorophenyl)-pyridinato-N,C2']picolinate), and the like. The "Piq" denotes 1-phenylisoquinoline, "acac" denotes acetylacetonate, and PPy denotes 2-phenylpyridine.

The compound for an organic photoelectric device according to one embodiment of the present invention has a thermal decomposition temperature (Td) ranging from 350 to 600 °C. Thereby, the compound for an organic photoelectric device according to one embodiment of the present invention has excellent thermal stability and may be used as a host material or a charge transport material. Therefore, life-span of the organic photoelectric device may be improved.

According to another embodiment of the present invention, an organic photoelectric device includes an anode, a cathode and an organic thin layer between the anode and the cathode, wherein the organic thin layer includes the compound for an organic photoelectric device according to one embodiment. The organic photoelectric device may include an organic light emitting diode, an organic solar cell, an organic transistor, organic photo conductor drum, an organic memory device, and the like. In case of an organic solar cell, the compound for an organic photoelectric device according to one embodiment may be applied to an electrode or an electrode buffer layer of an organic solar cell to improve quantum efficiency, or may be applied to an electrode material of a gate, source-drain electrodes, and the like of an organic transistor.

The organic thin layer including the compound for an organic photoelectric device may be an emission layer, a hole blocking layer, an electron blocking layer, an electron transport layer (ETL), an electron injection layer (EIL), a hole injection layer (HIL), a hole transport layer (HTL), or a combination thereof.

Hereinafter, an organic photoelectric device is illustrated in more detail.

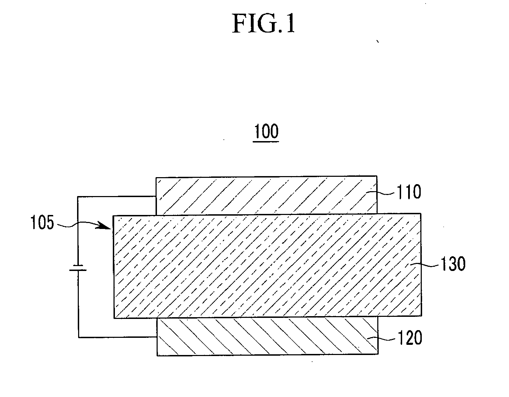

FIGS. 1 to 5 are cross-sectional views showing organic photoelectric devices including the compound for an organic photoelectric device.

Referring to FIGS. 1 to 5, the organic photoelectric devices 100, 200, 300, 400, and 500 according to one embodiment includes at least one organic thin layer 105 interposed between an anode 120 and a cathode 110.

A substrate of an organic photoelectric device is not particularly limited in the related arts, but a glass substrate or a transparent plastic substrate having excellent transparency, surface smoothness, handling ease, and water repellency.

The anode 120 includes an anode material laving a large work function to help hole injection into an organic thin layer. The anode material includes a metal such as nickel, platinum, vanadium, chromium, copper, zinc, gold, and the like or an alloy of the foregoing metals; metal oxide such as zinc oxide, indium oxide, indium tin oxide (ITO), indium zinc oxide (IZO), and the like; a combined metal and oxide such as ZnO/Al, SnO₂/Sb, and the like. But the anode material is not limited thereto. Particularly, the anode material may be a transparent electrode including ITO.

The cathode 110 includes a cathode material having a small work function to help electron injection into an organic thin layer. The cathode material includes a metal such as magnesium, calcium, sodium, potassium, titanium, indium, yttrium, lithium, gadolinium, aluminum, silver, tin, lead, cesium, barium, and the metal, or alloys thereof; or a multi-layered material such as LiF/Al, LiO₂/Al, LiF/Ca, LiF/Al, BaF₂/Ca, and the like. But the cathode material is not limited to the above materials. Particularly, the cathode material may be a metal electrode such as aluminum.

Referring to FIG. 1, the organic photoelectric device 100 includes an organic thin layer 105 including only an emission layer 130.

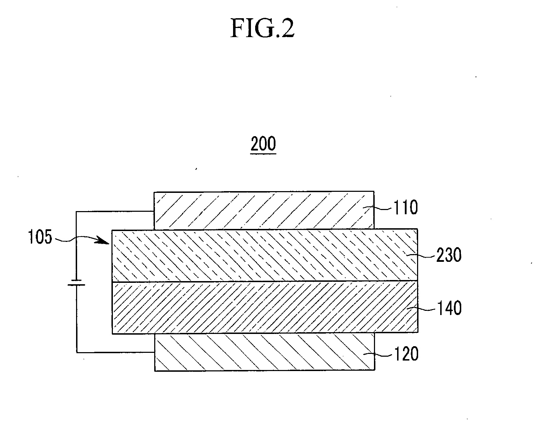

Referring to FIG. 2, a double-layered organic photoelectric device 200 includes an organic thin layer 105 including an emission layer 230 including an electron transport layer (ETL), and a hole transport layer (HTL) 140. The emission layer 130 also functions as an electron transport layer (ETL), and the hole transport layer (HTL) 140 layer has an excellent binding property with a transparent electrode such as ITO or an excellent hole transporting property.

The hole transport layer (HTL) 140 may include generally-used hole transport material without limitation, for example poly (3,4-ethylenedioxy-thiophene) (PEDOT) doped with poly(styrenesulfonate) (PSS) (PEDOT:PSS), N,N'-bis (3-methylphenyl)-N,N-diphenyl-[1,1'-biphenyl]-4,4'-diamine (TPD), N,N'-di (1-naphthyl)-N,N'-diphenylbenzidine (NPB) and the like, along with the compound for an organic photoelectric device according to one embodiment of the present invention. The hole transport material is not limited to the above materials.

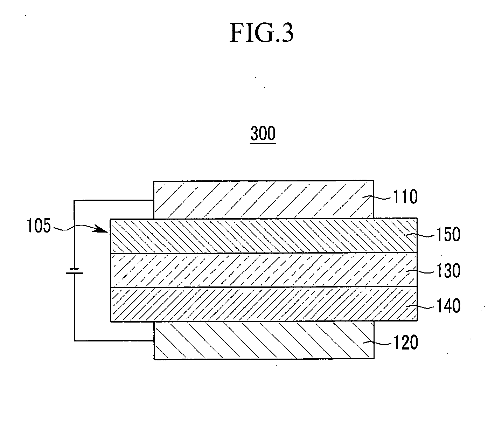

Referring to FIG. 3, a three-layered organic photoelectric device 300 includes an organic thin layer 105 including an electron transport layer (ETL) 150, an emission layer 130, and a hole transport layer (HTL) 140. The emission layer 130 is independently installed, and layers having an excellent electron transporting property or an excellent hole transporting property are separately stacked.

The electron transport layer (ETL) 150 may include generally-used electron transport material without limitation, for example, aluminum tris(8-hydroxyquinoline) (Alq₃); a 1,3,4-oxadiazole derivative such as 2-(4-biphenyl-5-phenyl-1,3,4-oxadiazole (PBD); a quinoxalin derivative such as 1,3,4-tris[(3-phenyl-6-trifluoromethyl) quinoxalin-2-yl]benzene (TPQ); and a triazole derivative, along with the compound for an organic photoelectric device according to one embodiment of the present invention. The electron transport material is not limited to the above materials.

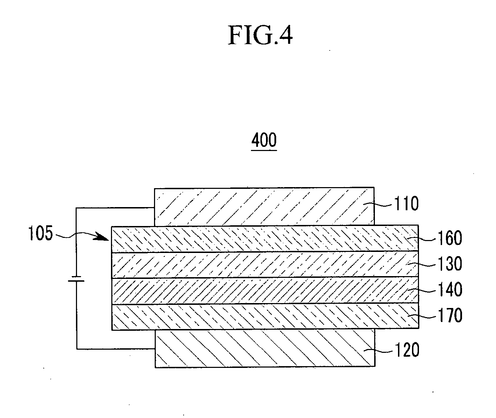

FIG. 4 shows a four-layered organic photoelectric device 400 that includes an organic thin layer 105 including an electron injection layer (EIL) 160, an emission layer 130, a hole transport layer (HTL) 140, and a hole injection layer (HIL) 170 for binding with the cathode of ITO.

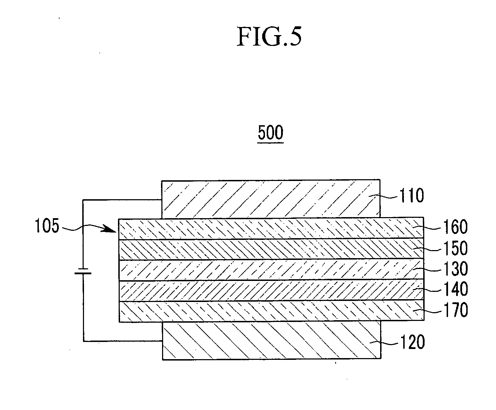

FIG. 5 shows a five layered organic photoelectric device 500 that includes an organic thin layer 105 including an electron transport layer (ETL) 150, an emission layer 130, a hole transport layer (HTL) 140, and a hole injection layer (HIL) 170, and further includes an electron injection layer (EIL) 160 to achieve a low voltage.

The emission layers 130 and 230 may have a thickness of 5 to 1000 nm, the hole transport layer (HTL) 140 and electron transport layer (ETL) 150 may have a thickness of 10 to 10,000 Å, respectively. However, the thicknesses are not limited to the above range.

In FIGS. 1 to 5, the organic thin layer 105 selected from the electron transport layer (ETL) 150, electron injection layer (EIL) 160, emission layer 130 and 230, hole transport layer (HTL) 140, hole injection layer (HIL) 170 and a combination thereof includes the compound for an organic photoelectric device according to one embodiment. The material for the organic photoelectric device may be used for an electron transport layer (ETL) 150 including the electron transport layer (ETL) 150 or electron injection layer (EIL) 160. When it is used for the electron transport layer (ETL), it is possible to provide an organic photoelectric device having a simpler structure because it does not require an additional hole blocking layer.

Furthermore, when the compound for an organic photoelectric device is included in the emission layer 130 and 230, the material for the organic photoelectric device may be included as a phosphorescent host, and the emission layer 130 and 230 may further include a dopant. The dopant may be a red, green and blue, or white phosphorescent dopant.

The organic photoelectric device may be fabricated by: forming an anode on a substrate, forming an organic thin layer in accordance with a dry coating method such as evaporation, sputtering, plasma plating, and ion plating, or a wet coating method such as spin coating, dipping, and flow coating; and providing a cathode thereon.

Another embodiment of the present invention provides a display device including the organic photoelectric device.

Hereinafter, the embodiments are illustrated in more detail with reference to examples. However, the following are exemplary embodiments and are not limiting.

### Synthesis of Compound for Organic photoelectric device

### Example 1

A compound for an organic photoelectric device was synthesized according to the following reaction scheme 1.

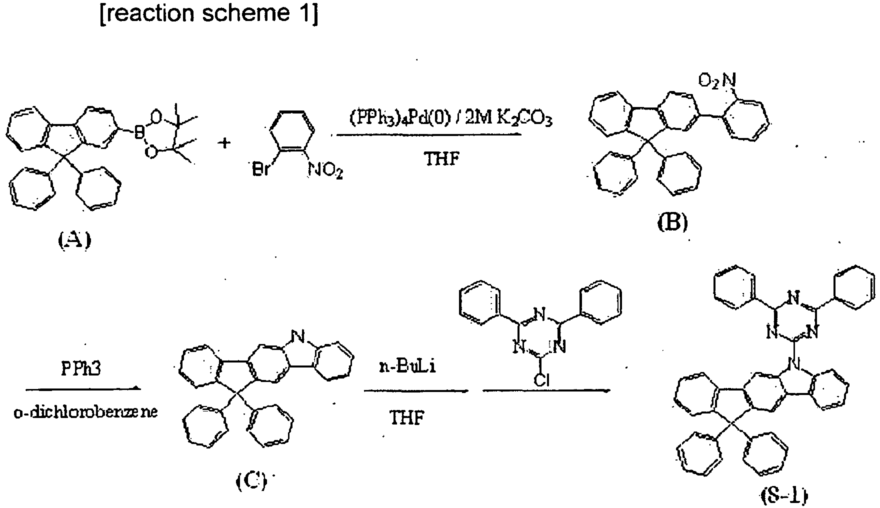

### First Step: Synthesis of Intermediate product (B)

11.0 g (24.7 mmol) of the compound A, 6.0 g (29.7 mmol) of 1-bromo-2-nitro benzene, 1 g (0.86 mmol) of tetrakistriphenylphosphine palladium were dissolved in 200 mL of tetrahydrofuran (THF) in a 500 mL round-bottomed flask with a thermometer, a reflux-condenser, and an agitator under an argon atmosphere, and 50 mL of 2M potassium carbonate was added thereto. The mixture was agitated at 75 °C for 24 hours.

The agitated reactant was cooled down to a room temperature to complete the reaction and then, extracted with methylene chloride and cleaned with water. Next, the reactant was treated with anhydrous magnesium sulfate to remove moisture and filtrated to remove an organic solvent therein. The final residue was purified through silica gel chromatography using a mixed solvent prepared by mixing methylene chloride and hexane in a volume ratio of 1:1, obtaining 9 g of an intermediate product (B) (a yield: 82.7 %).

### Second Step: Synthesis of Intermediate product (C)

8 g (18.2 mmol) the intermediate product (B) synthesized in the first step and 14.3 g (54.6 mmol) of triphenylphosphine were dissolved in 150 ml of dichlorobenzene. The solution was heated and refluxed at 160 °C under an argon atmosphere.

*The reactant was extracted with methylenechloride and cleaned with water, after the organic solvent therein was distillated and removed under reduced pressure. Then, the reactant was treated with anhydrous magnesium sulfate to remove moisture and filtrated to remove an organic solvent therein. The final residue was purified through silica gel chromatography using a mixed solvent prepared by mixing methylenechloride and hexane in a volume ratio of 2:1, obtaining 5.3 g of an intermediate product (C) (a yield: 71.5 %).

### Third Step: Synthesis of Compound for Organic photoelectric device

5 g (12.2 mmol) of the intermediate product (C) synthesized in the second step was dissolved in 100 mL of anhydrous tetrahydrofuran (THF), and 9.2 mL of 1.6 M n-BuLi was added in a dropwise fashion at -78 °C. The mixture was slowly agitated for 30 minutes. Next, the reactant was further agitated at a room temperature for 20 minutes and then, mixed with 3.59 g (13.4 mmol) of 2-chloro-4,6-diphenyl triazine at -78 °C. The mixture was agitated at a room temperature for 12 hours.

The agitated reactant was cooled down to a room temperature to complete the reaction and then, extracted with methylene chloride and cleaned with water. Then, the resulting reactant was treated with anhydrous magnesium sulfate to remove moisture and filtrated to remove an organic solvent therein. The final residue was purified and recrystallized through silica gel chromatography using a mixed solvent prepared by mixing methylene chloride and hexane in a volume ratio of 1:3, obtaining 4 g of a compound for an organic photoelectric device (a yield: 51.3 %).

The compound for an organic photoelectric device was performed regarding atomic analysis.
Calcd: C, 86.49; H, 4.73; N, 8.77
Found: C, 86.50; H, 4.72; N, 8.77

### Example 2

A compound for an organic photoelectric device was synthesized according to the following reaction scheme 2.

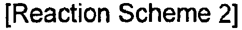

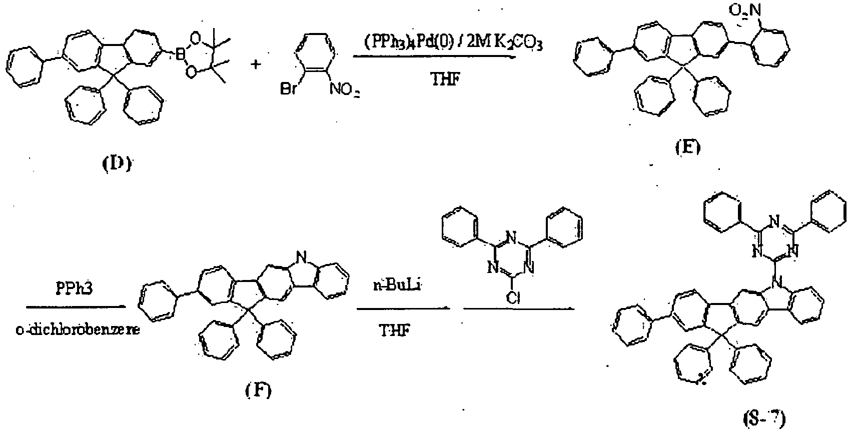

### First Step: Synthesis of Intermediate product (E)

10.0 g (19.2 mmol) of a compound B, 4.7 g (23.2 mmol) of 1-bromo-2-nitro benzene, and 0.8 g (0.69 mmol) of tetrakistriphenylphosphinepalladium were dissolved in 200 mL of tetrahydrofuran in a 500 mL round-bottomed flask with a thermometer, a reflux-condenser, and an agitator, and 50 mL of tetratriethyl ammonium hydroxide with a concentration of 20% was added thereto under an argon atmosphere. The mixture was agitated at 75 °C for 24 hours.

The resulting reactant was cooled down to a room temperature to complete the reaction and then, extracted with methylene chloride and cleaned with water. Then, the reactant was treated with anhydrous magnesium sulfate and filtrated to remove an organic solvent therein. The final residue was purified through silica gel chromatography using a mixed solvent prepared by mixing methylenechloride and hexane in a volume ratio of 1:1, obtaining 7 g of an intermediate product (E) (a yield: 72 %).

### Second Step: Synthesis of Intermediate product (F)

7 g (13.5 mmol) of the intermediate product (E) synthesized in the first step and 10.6 g (40.7 mmol) of triphenylphosphine were dissolved in 150 ml of dichlorobenzene. The mixture was heated and refluxed at 160 °C under an argon atmosphere.

The reactant was distillated to remove an organic solvent under a reduced pressure and then, extracted with methylene chloride and cleaned with water. Next, the reactant was treated with anhydrous magnesium sulfate to remove moisture and filtrated to remove an organic solvent therein. The final residue was purified through silica gel chromatography using a mixed solvent prepared by mixing methylenechloride and hexane in a volume ratio of 2:1, obtaining 4.3 g of an intermediate product (F) (a yield: 65.9 %).

### Third Step: Synthesis of Compound for Organic photoelectric device

4 g (8.27 mmol) of the intermediate product (F) synthesized in the second step was dissolved in 100 mL of anhydrous tetrahydrofuran (THF), and 6.2 mL of 1.6 M n-BuLi was slowly added thereto in a dropwise fashion at -78 °C.

The mixture was agitated for 30 minutes. Then, the mixture was further agitated at a room temperature for 20 minutes, and 2.43 g (9.09 mmol) of 2-chloro-4,6-diphenyl triazine was added thereto at -78 °C. The mixture was agitated at a room temperature for 12 minutes.

The reactant was cooled down to a room temperature to complete the reaction and then, extracted with methylene chloride and cleaned with water. Then, the reactant was treated with anhydrous magnesium sulfate to remove moisture and filtrated to remove an organic solvent therein. The final residue was purified and recrystallized through silica gel chromatography using a mixed solvent prepared by mixing methylenechloride and hexane in a volume ratio of 1:3, obtaining 3.2 g of a compound for an organic photoelectric device (a yield: 54.1 %).

The compound for an organic photoelectric device was performed regarding atomic analysis. The results are provided as follows.
Calcd: C, 86.37; H, 4.79; N, 7.84
Found: C, 86.36; H, 4.80; N, 7.84

### Preparation of Organic light emitting diode

### Example 3

The compound synthesized according to Example 1 as a host and Ir(PPy)₃ as a dopant were used to fabricate an organic light emitting diode. Herein, ITO was formed to be 1000 Å thick as an anode, while aluminum (AI) was formed to be 1500 Å thick as a cathode.

In particular, an anode for an organic light emitting diode was fabricated by cutting an ITO glass substrate with 15 Ω/cm² of sheet resistance to have a size of 50 mm × 50 mm × 0.7 mm and then, ultrasonic wave cleaning it in acetone and isopropyl alcohol and pure water respectively for 15 minutes and UV ozone-cleaning it for 30 minutes.

On the substrate, a 800 Å hole transport layer (HTL) was formed by depositing N,N'-di(1-naphthyl)-N,N'-diphenylbenzidine (NPB) (70 nm) and 4,4',4"-tri(N-carbazolyl)triphenylamine (TCTA) (10 nm) with a vacuum degree of 650×10⁻⁷ Pa at a deposit speed ranging from 0.1 to 0.3 nm/s.

Then, a 300 Å-thick emission layer was formed by using the compound synthesized according to Example 1 under the same vacuum deposition condition, and a phosphorescent dopant, Ir(PPy)₃, was simultaneously deposited. Herein, the phosphorescence dopant was deposited in an amount of 7 wt% based on 100 wt% of the total weight of the emission layer by regulating its deposition speed.

On the emission layer, a 50 Å-thick hole-blocking layer was formed by depositing aluminum(III) bis(2-methyl-8-quinolinato)4 -phenylphenolate (BAlq) under the same vacuum deposition condition.

Then, a 200 Å-thick electron transport layer (ETL) was formed thereon by depositing Alq₃ under the same vacuum deposition condition.

On the electron transport layer (ETL), LiF and Al were sequentially deposited to form a cathode, fabricating an organic light emitting diode.

The organic light emitting diode had a structure of ITO/ NPB (70 nm)/ TCTA (10 nm)/ EML (the compound (93 wt%) of Example 1 + Ir(PPy)₃ (7 wt%), 30 nm)/ Balq (5 nm)/ Alq₃ (20 nm)/ LiF (0.5 nm) / Al (150 nm).

### Comparative Example 1

An organic light emitting diode was fabricated according to the same method as Example 3 except for using 4,4-N,N-dicarbazolebiphenyl (CBP) as a host for an emission layer instead of the compound of Example 1.

### Experimental Example 1: Performance Evaluation of Organic light emitting diode

The organic light emitting diodes according to Example 3 and Comparative Example 1 were measured regarding current density change and luminance change depending on voltage and luminous efficiency. In particular, the current density change and luminance change depending on voltage and luminous efficiency were measured in the following method. The results are provided in the following Table 1.

### (1) Current density change depending on voltage change

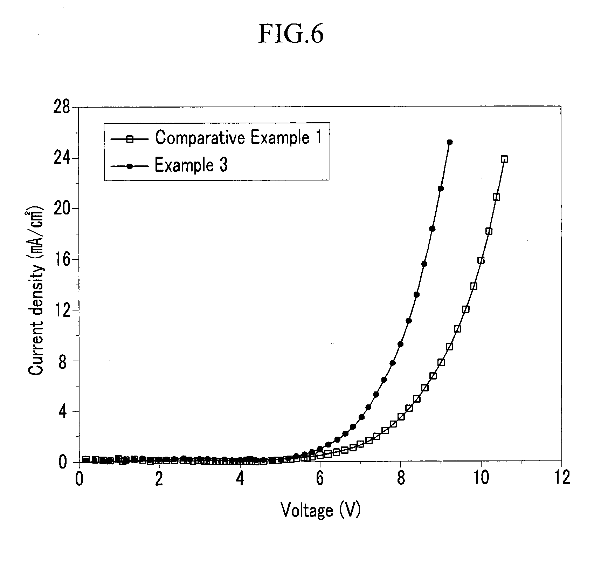

The organic light emitting diodes were measured regarding current using a current-voltage meter (Keithley 2400) while their voltages were increased from 0 V to 10 V. The results are provided in FIG. 6.

### (2) Luminance change depending on voltage change

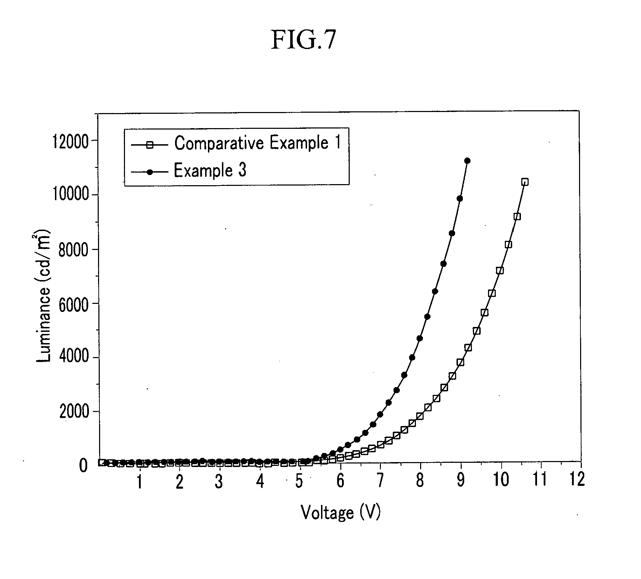

The organic light emitting diodes were measured regarding luminance using a luminance meter (Minolta Cs-1000A), while their voltages were increased from 0 V to 10 V. The results are provided in FIG. 7.

### (3) Luminous efficiency

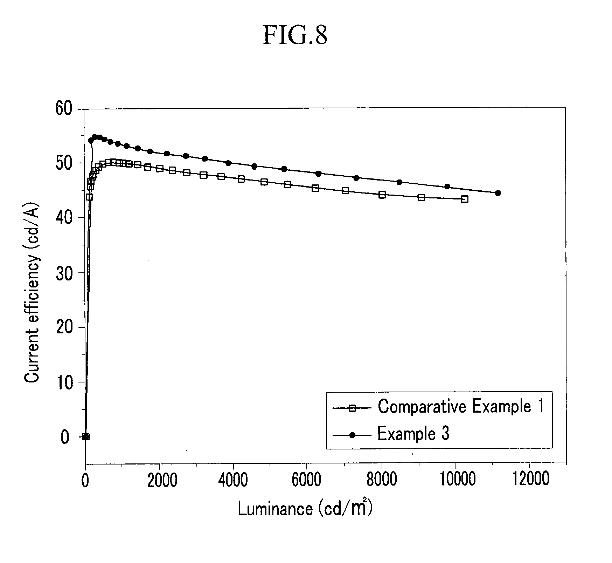

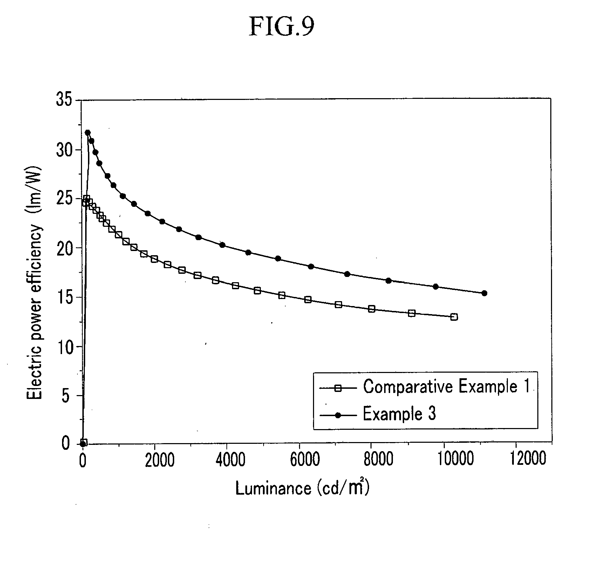

The luminance and current density obtained in the above (1) and (2) and voltage were used to calculate current efficiency (cd/A) and electric power efficiency (Im/W) at the same luminance (2000 cd/m²). The result are provided in FIG. 8 and 9.

### (4) Color coordinate

The organic light emitting diodes were measured regarding color coordinate using a luminance meter (Minolta Cs-100A). The results are provided in the following Table 1.

**(Table 1)**

| Device | Host material of emission layer | at 2000 cd/m² | | | |
|---|---|---|---|---|---|
| | | Driving voltage (\/) | Current efficiency (cd/A) | Electric power efficiency (lm/W) | Color coordinate (x,y) |
| Example 3 | Example 1 | 7.2 | 51.8 | 22.6 | 0.308, 0.622 |
| Comparative Example 1 | CBP | 8.2 | 49.2 | 18.5 | 0.295, 0.622 |

Referring to Table 1, the organic light emitting diode of Example 3 had about 1 V lower driving voltage than the one of Comparative Example 1 and very improved current efficiency and electric power efficiency compared therewith. Accordingly, the compound according to Example 1 was identified to lower driving voltage and to improve luminance and efficiency of an organic light emitting diode.

## Claims

1. A compound for an organic photoelectric device in which substituents represented by the following Chemical Formulas 1 to 3 are sequentially combined: 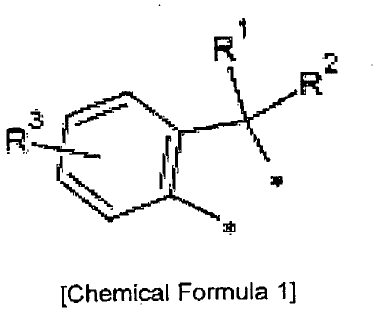 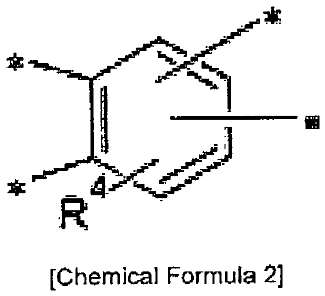 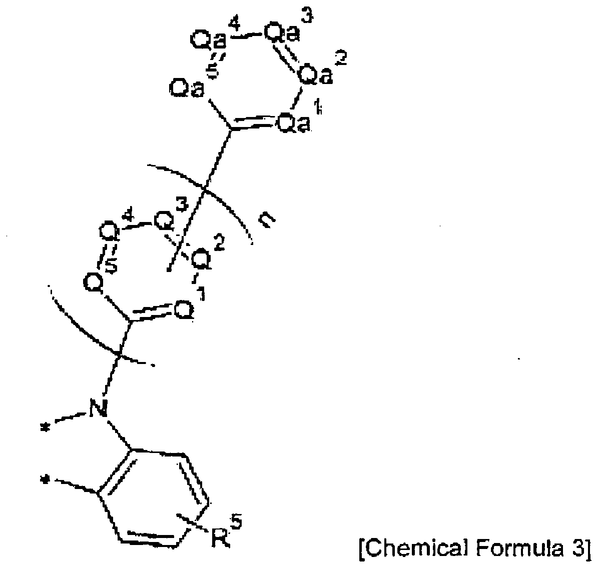 wherein, in Chemical Formulas 1 to 3,
R¹ and R² are the same or different and are each independently hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof, R³ to R⁵ are the same or different and are each independently hydrogen, a C1 to C30 alkyl group, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof, and
n is an integer ranging from 0 to 5.
and wherein in Chemical formula 3 Q¹ to Q⁵ and Qa¹ to Qa⁵ are the same or different and are each independently N or CR,
provided that one or more of Qa¹, Qa³ and Qa⁵ is N,
the rest thereof are each independently CR, and wherein the R is hydrogen, a substituted or unsubstituted C6 to C30 aryl group, a substituted or unsubstituted C3 to C30 heteroaryl group, or a combination thereof.

2. The compound for an organic photoelectric device of claim 1, wherein the Chemical Formula 1 represented by the following Chemical Formula 1a: 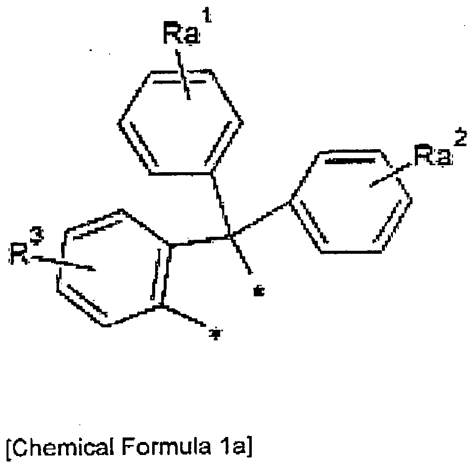 wherein, in ChemicalFormula 1a,
Ra¹ and Ra² are the same or different and are each independently hydrogen, a substituted or unsubstituted carbazolyl group, a substituted or unsubstituted C6 to C30 aryl group, a substituted or unsubstituted C6 to C30 arylamine group, or a combination thereof, and
R³ is hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof.

3. The compound for an organic photoelectric device of claim 1 or 2, wherein the Chemical Formula 2 is represented by the following Chemical Formula 2a or 2b: 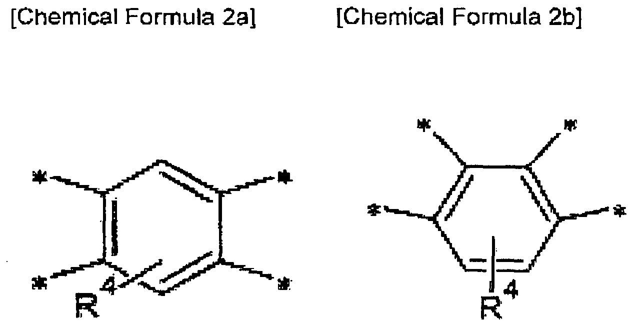 wherein , in Chemical Formulas 2a and 2b,
R⁴ is hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof.

4. The compound for an organic photoelectric device of any of the claims 1 to 3,
wherein Q¹ to Q⁵ and Qa¹ to Qa⁵ in Chemical Formula 3 are the same or different and are each independently N or CR,
provided that one to three selected from Q¹ to Q⁵ is N,
one to three selected from Qa1 to Qa5 is N, and
the rest of them are each independently CR, wherein the R is hydrogen, a substituted or unsubstituted C6 to C30 aryl group, a substituted or unsubstituted C3 to C30 heteroaryl group, or a combination thereof.

5. The compound for an organic photoelectric device of any of the claims 1 to 4, wherein n in Chemical Formula 3 is 0 to 2.

6. The compound for an organic photoelectric device of any of the claims 1 to 5, wherein the Chemical Formula 3 is represented by the following Chemical Formula 3a: 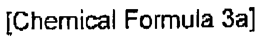 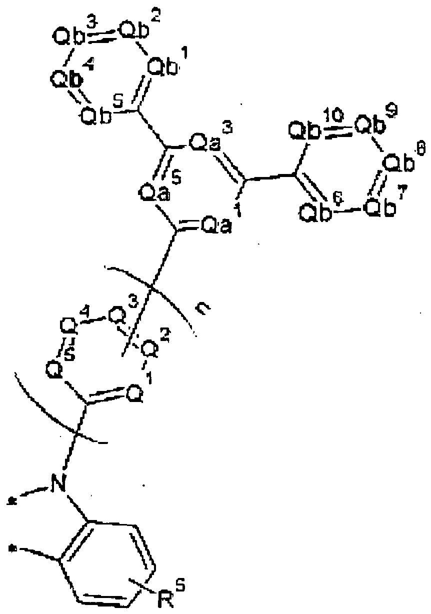 wherein, in Chemical Formula 3a,
Qa¹, Qa³ and Qa⁵ are the same or different, and are each independently N or CH, provided that one or more of Qa¹, Qa³ and Qa⁵ is N,
Q¹ to Q⁵ and Qb¹ to Qb¹⁰ are the same or different and are each independently N or CR, wherein R is hydrogen, a substituted or unsubstituted C6 to C30 aryl group, a substituted or unsubstituted C3 to C30 heteroaryl group, or a combination thereof,
R⁵ is hydrogen, a carbazolyl group, a substituted or unsubstituted C6 to C30 aryl group, a substituted or unsubstituted C3 to C30 heteroaryl group, a substituted or unsubstituted C6 to C30 arylamine group, or a combination thereof, and
n is an integer ranging from 0 to 5.

7. The compound for an organic photoelectric device of any claims 1 to 6, wherein in Chemical Formulas 1 to 3, R³ to R⁵ are the same or different and are each independently hydrogen, a C1 to C30 alkyl group, a C6 to C30 aryl group, or a combination thereof.

8. The compound for an organic photoelectric device of any of the claims 1 to 7, wherein the compound for an organic photoelectric device is represented by the following Chemical Formulas 4 to 9: 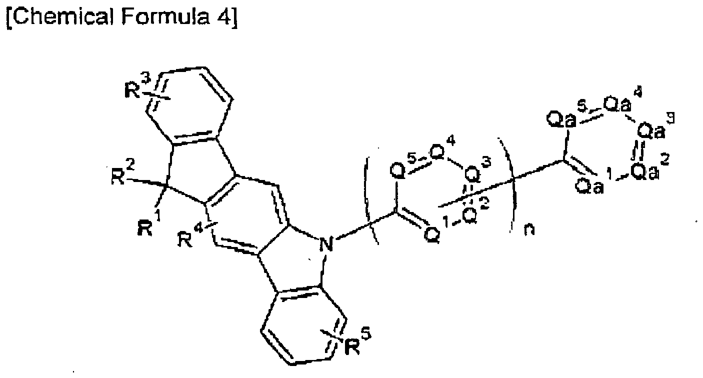 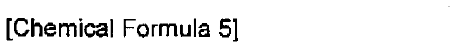 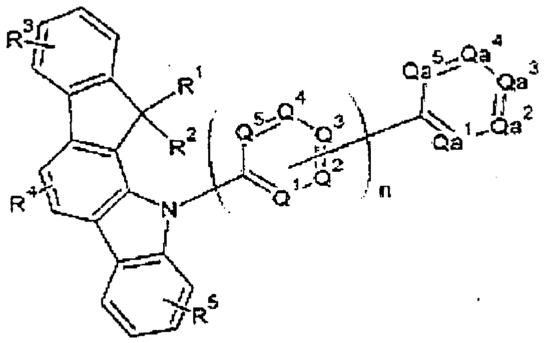 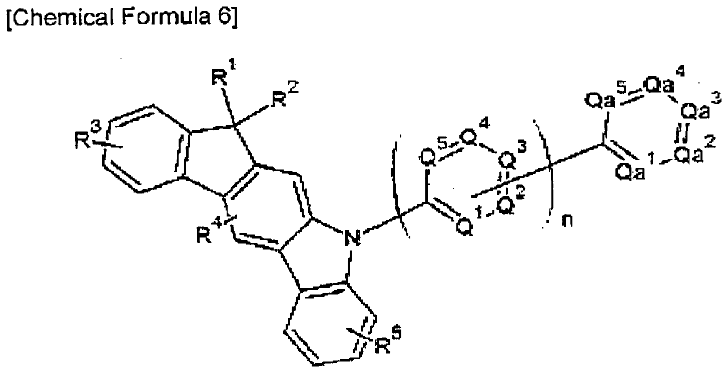 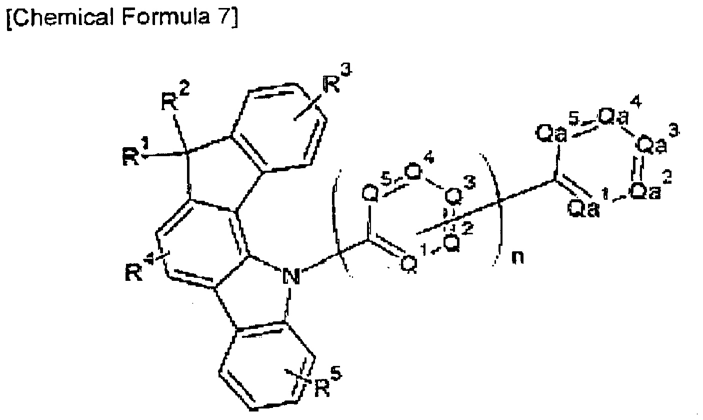 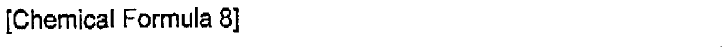 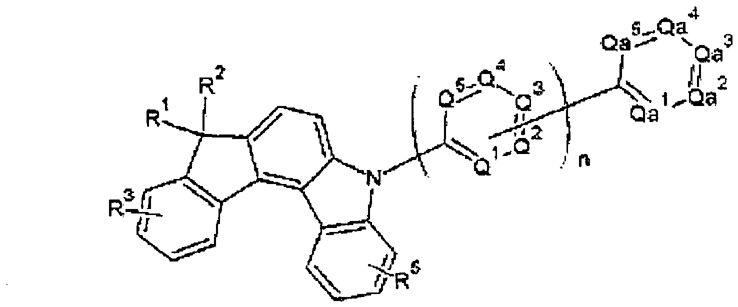 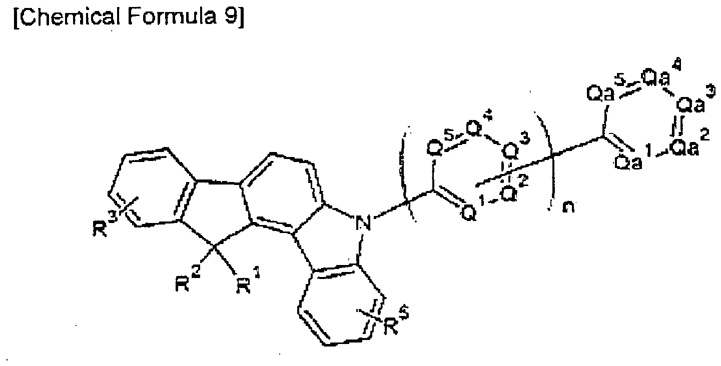 wherein, in Chemical Formulas 4 to 9,
Q¹ to Q⁵ and Qa¹ to Qa⁵ are the same or different and are each independently N or CR, wherein R is hydrogen, a substituted or unsubstituted C6 to C30 aryl group, a substituted or unsubstituted C3 to C30 heteroaryl group, or a combination thereof,
R¹ to R⁵ are the same or different and are each independently hydrogen, a substituted or unsubstituted carbazolyl group, a substituted or unsubstituted C6 to C30 aryl group, a substituted or unsubstituted C3 to C30 heteroaryl group, a substituted or unsubstituted C6 to C30 arylamine group, or a combination thereof, and
n is an integer ranging from 0 to 5.

9. The compound for an organic photoelectric device of any of the claims 1 to 8, wherein the compound for an organic photoelectric device is one represented by the following Chemical Formulas 10 to 33:

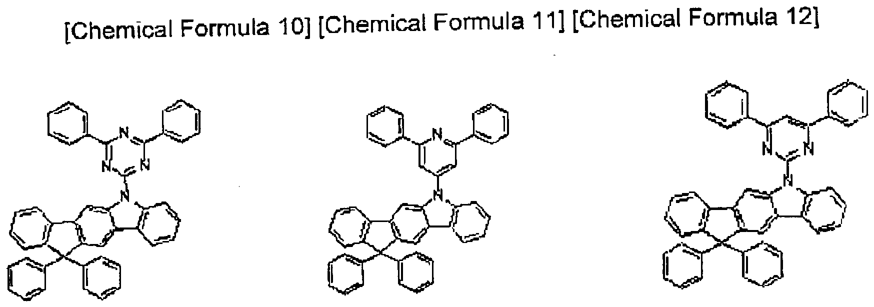

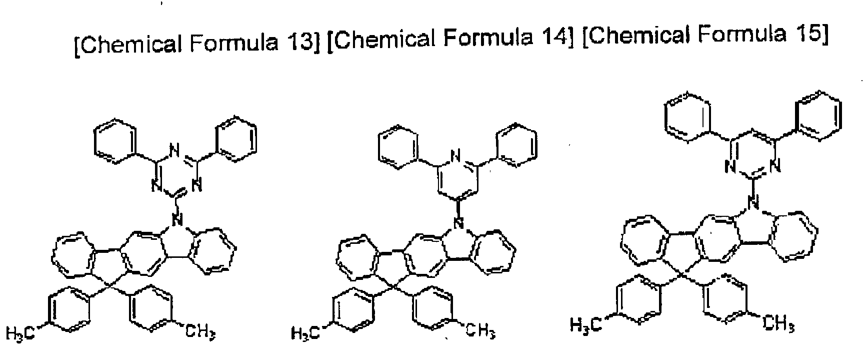

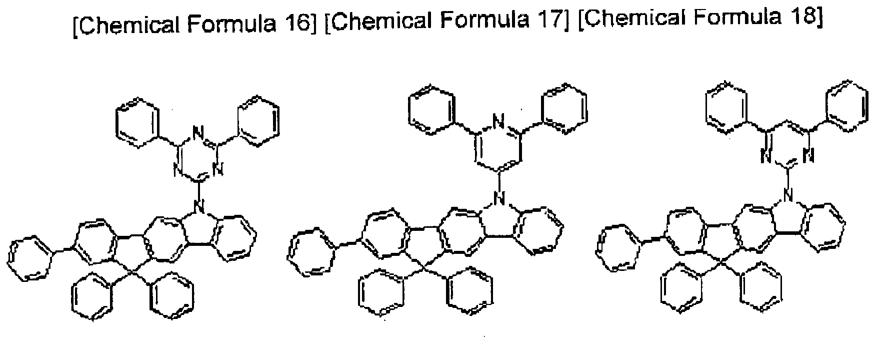

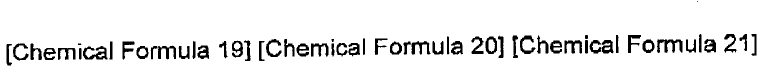

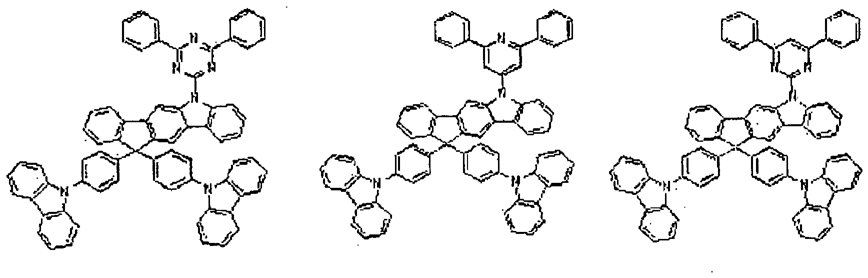

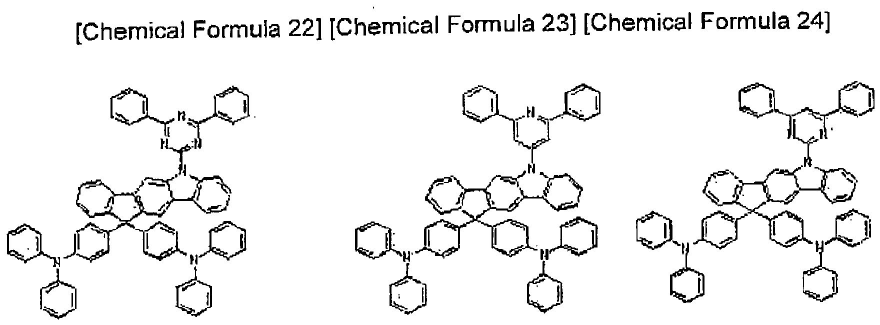

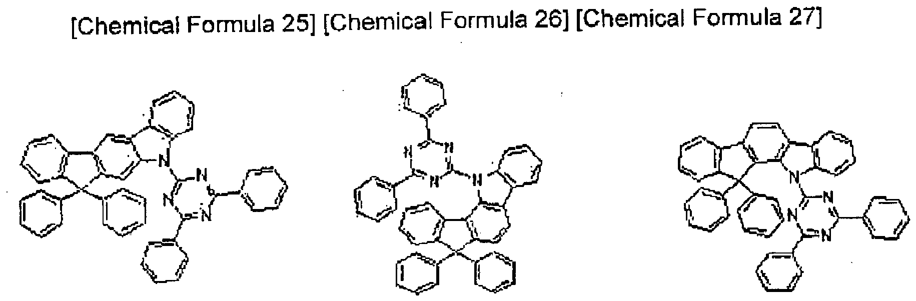

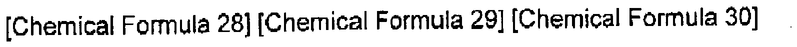

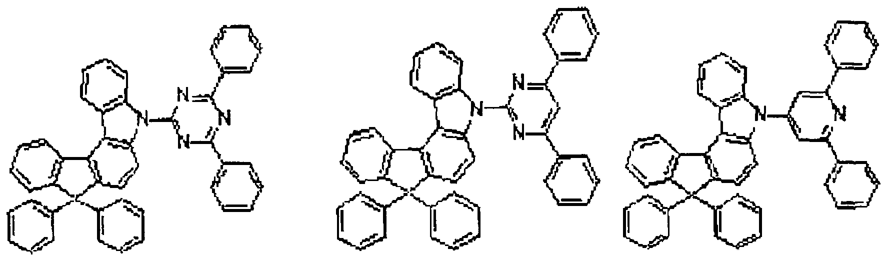

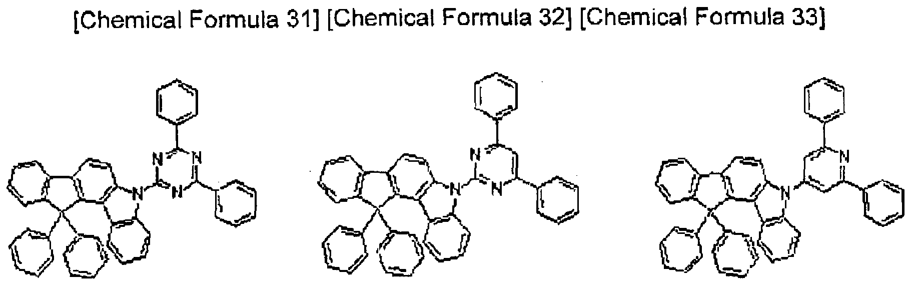

10. The compound for an organic photoelectric device of any of the claims 1 to 9, wherein the compound for an organic photoelectric device has a thermal decomposition temperature (Td) of 350° C to 600 °C

11. Use of a compound according to any of the claims 1 to 10 as a charge transport material or a host material in organic photoelectric devices.

12. An organic photoelectric device comprising
an anode, a cathode, and at least one organic thin layer interposed between the anode and cathode,
wherein at least one organic thin layer includes the compound for an organic photoelectric device according to one of claim 1 to 10.

13. The organic photoelectric device of claim 12, wherein the organic thin layer is an emission layer, a hole blocking layer, an electron blocking layer, an electron transport layer (ETL), an electron injection layer (EIL), a hole injection layer (HIL), a hole transport layer (HTL), or a combination thereof.

14. A display device comprising the organic photoelectric device according to claim 12.

## Patentansprüche

1. Eine Verbindung für eine organisch photoelektrische Vorrichtung in welcher Substituenten, wiedergegeben durch die folgenden chemischen Formeln 1 bis 3, sequentiell verbunden werden: 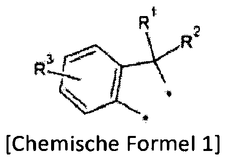 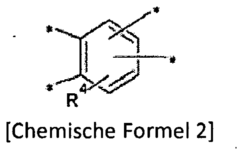 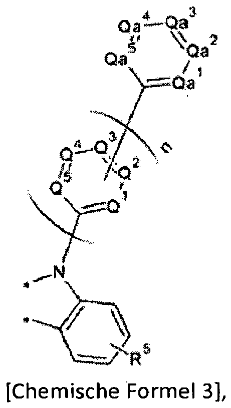 wobei in den chemischen Formeln 1 bis 3,
R¹ und R² dieselben oder unterschiedlich und jedes unabhängig voneinander Wasserstoff, eine substituierte oder nicht substituierte Carbazolyl-Gruppe, eine substituierte oder nicht substituierte C6 bis C30 Aryl-Gruppe, eine substituierte oder nicht substituierte C3 bis C30 Heteroaryl-Gruppe, eine substituierte oder nicht substituierte C6 bis C30 Arylamin-Gruppe, oder eine Kombination daraus ist, R3 bis R5 dieselben oder unterschiedlich und jedes unabhängig voneinander Wasserstoff, eine C1 bis C30 Alkyl-Gruppe, eine substituierte oder nicht substituierte Carbazolyl-Gruppe, eine substituierte oder nicht substituierte C6 bis C30 Aryl-Gruppe, eine substituierte oder nicht substituierte C3 bis C30 Heteroaryl-Gruppe, eine substituierte oder nicht substituierte C6 bis C30 Arylamin-Gruppe, oder eine Kombination daraus ist, und
n ist eine ganze Zahl von 0 bis 5;
und wobei in der chemischen Formel 3 Q¹ bis Q⁵ und Qa¹ bis Qa⁵ dieselben oder unterschiedlich,und jedes unabhängig voneinander N oder CR ist,
vorausgesetzt, dass einer oder mehrere von Qa¹, Qa³ und Qa⁵ Stickstoff ist,
der Rest davon ist jeweils unabhängig voneinander CR, und wobei das R Wasserstoff, eine substituierte oder nicht substituierte C6 bis C30 Aryl-Gruppe, eine substituierte oder nicht substituierte C3 bis C30 Heteroaryl-Gruppe, oder eine Kombination daraus ist.

2. Die Verbindung für eine organisch photoelektrische Vorrichtung nach Anspruch 1, wobei die chemische Formel 1 durch folgende chemische Formel 1a wiedergegeben wird: 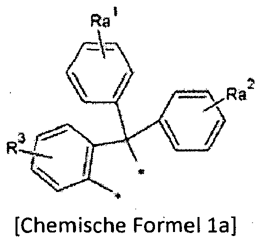 wobei, in der chemischen Formel 1a,
Ra¹ und Ra² dieselben oder unterschiedlich und jedes unabhängig voneinander Wasserstoff, eine substituierte oder nicht substituierte Carbazolyl-Gruppe, eine substituierte oder nicht substituierte C6 bis C30 Aryl-Gruppe, eine substituierte oder nicht substituierte C6 bis C30 Arylamin-Gruppe, oder eine Kombination daraus ist, und
R³ Wasserstoff, eine substituierte oder nicht substituierte Carbazolyl-Gruppe, eine substituierte oder nicht substituierte C6 bis C30 Aryl-Gruppe, eine substituierte oder nicht substituierte C3 bis C30 Heteroaryl-Gruppe, eine substituierte oder nicht substituierte C6 bis C30 Arylamin-Gruppe, oder eine Kombination daraus ist

3. Die Verbindung für eine organisch photoelektrische Vorrichtung nach Anspruch 1 oder 2, wobei die chemische Formel 2 durch die folgenden chemischen Formeln 2a oder 2b wiedergegeben wird: 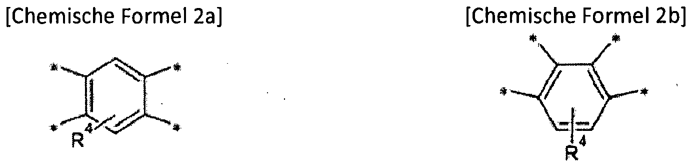 wobei, in den chemischen Formeln 2a und 2b,
R⁴ Wasserstoff, eine substituierte oder nicht substituierte Carbazolyl-Gruppe, eine substituierte oder nicht substituierte C6 bis C30 Aryl-Gruppe, eine substituierte oder nicht substituierte C3 bis C30 Heteroaryl-Gruppe, eine substituierte oder nicht substituierte C6 bis C30 Arylamin-Gruppe, oder eine Kombination daraus ist.

4. Die Verbindung für eine organisch photoelektrische Vorrichtung nach einem der Ansprüche 1 bis 3, wobei Q¹ bis Q⁵ und Qa¹ bis Qa⁵ in der chemischen Formel 3 dieselben oder unterschiedlich sind und jeweils unabhängig N oder CR,
vorausgesetzt das einer bis drei ausgewählt aus Q¹ bis Q⁵ ist N,
einer bis drei ausgewählt aus Qa¹ bis Qa⁵ ist N, und
der Rest von ihnen jeweils unabhängig voneinander CR sind, wobei das R Wasserstoff, eine substituierte oder nicht substituierte C6 bis C30 Aryl-Gruppe, eine substituierte oder nicht substituierte C3 bis C30 Heteroaryl-Gruppe, oder eine Kombination daraus ist.

5. Die Verbindung für eine organisch photoelektrische Vorrichtung nach einem der Ansprüche 1 bis 4, wobei n in der chemischen Formel 3 0 bis 2 ist.

6. Die Verbindung für eine organisch photoelektrische Vorrichtung nach einem der Ansprüche 1 bis 5, wobei die chemische Formel 3 durch folgende chemische Formel 3a wiedergegeben wird: 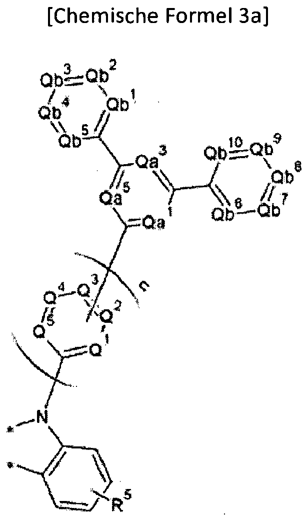 wobei in der chemischen Formel 3a,
Qa¹, Qa³ und Qa⁵ dieselben oder unterschiedlich, und jeweils unabhängig voneinander N oder CH sind, vorausgesetzt das einer oder mehrere der Qa¹, Qa³ und Qa⁵ ist N,
Q¹ bis Q⁵ und Qb¹ bis Qb¹⁰ dieselben oder unterschiedlich, und jeweils unabhängig voneinander N oder CR sind, wobei R Wasserstoff, eine substituierte oder nicht substituierte C6 bis C30 Aryl-Gruppe, eine substituierte oder nicht substituierte C3 bis C30 Heteroaryl-Gruppe, oder eine Kombination daraus ist,
R⁵ ist Wasserstoff, eine Carbazolyl-Gruppe, eine substituierte oder nicht substituierte C6 bis C30 Aryl-Gruppe, eine substituierte oder nicht substituierte C3 bis C30 Heteroaryl-Gruppe, eine substituierte oder nicht substituierte C6 bis C30 Arylamin-Gruppe, oder eine Kombination daraus ist, und
n ist eine ganze Zahl von 0 bis 5.

7. Die Verbindung für eine organisch photoelektrische Vorrichtung nach einem der Ansprüche 1 bis 6, wobei in den chemischen Formeln 1 bis 3, R³ bis R⁵ dieselben oder unterschiedlich, und jeweils unabhängig voneinander Wasserstoff, eine C1 bis C30 Alkyl-Gruppe, eine C6 bis C30 Aryl-Gruppe, oder eine Kombination daraus sind.

8. Die Verbindung für eine organisch photoelektrische Vorrichtung nach einem der Ansprüche 1 bis 7, wobei die Verbindung für eine organisch photoelektrische Vorrichtung durch folgende chemische Formeln 4 bis 9 wiedergegeben wird: 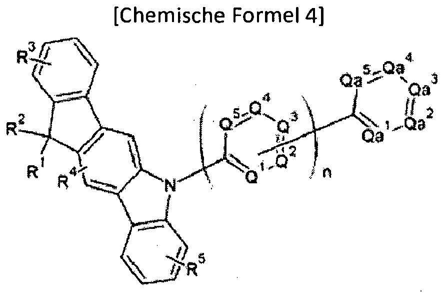 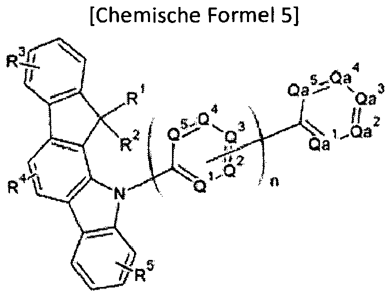 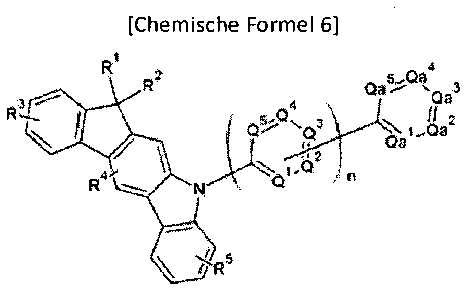 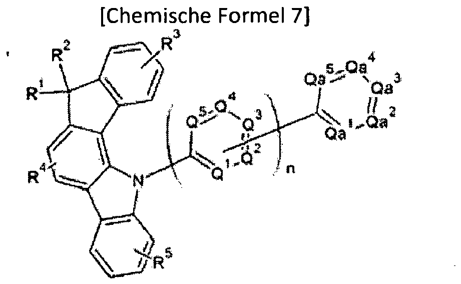 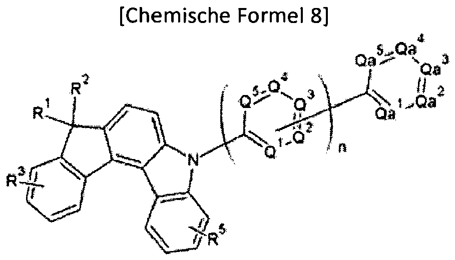 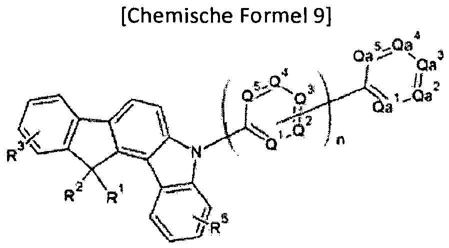 wobei, in den chemischen Formeln 4 bis 9,
Q¹ bis Q⁵ und Qa¹ bis Qa⁵ dieselben oder unterschiedlich und jeweils unabhängig voneinander N oder CR sind, wobei das R Wasserstoff, eine substituierte oder nicht substituierte C6 bis C30 Aryl-Gruppe, eine substituierte oder nicht substituierte C3 bis C30 Heteroaryl-Gruppe, oder eine Kombination daraus ist,
R¹ bis R⁵ dieselben oder unterschiedlich und jedes unabhängig voneinander Wasserstoff, eine substituierte oder nicht substituierte Carbazolyl-Gruppe, eine substituierte oder nicht substituierte C6 bis C30 Aryl-Gruppe, eine substituierte oder nicht substituierte C3 bis C30 Heteroaryl-Gruppe, eine substituierte oder nicht substituierte C6 bis C30 Arylamin-Gruppe, oder eine Kombination daraus ist, und
n ist eine ganze Zahl von 0 bis 5.

9. Die Verbindung für eine organisch photoelektrische Vorrichtung nach einem der Ansprüche 1 bis 8, wobei die Verbindung für eine organisch photoelektrische Vorrichtung durch eine der folgenden chemischen Formeln 10 bis 33 wiedergegeben wird:

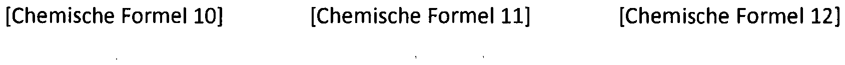

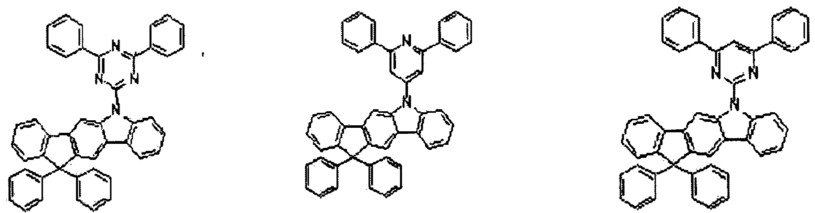

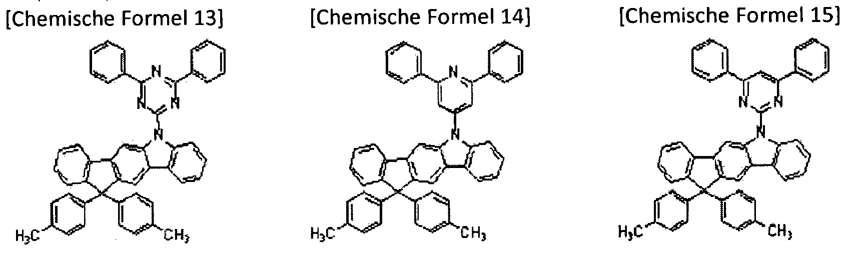

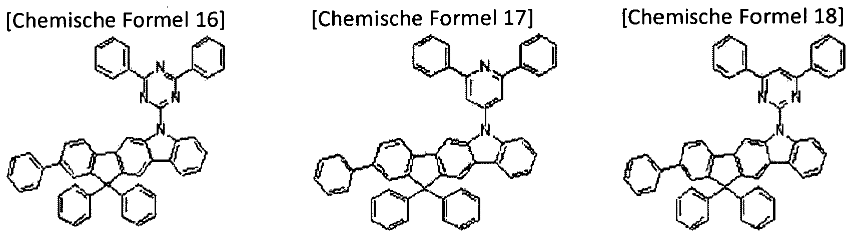

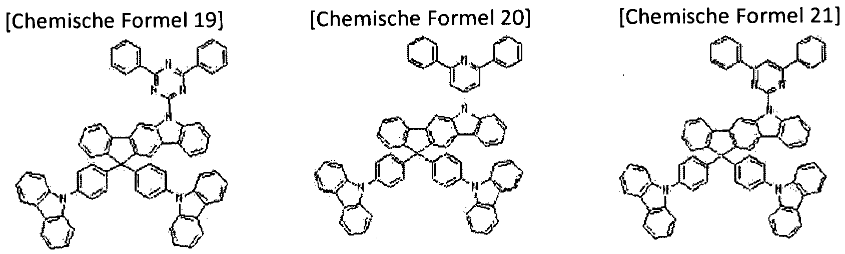

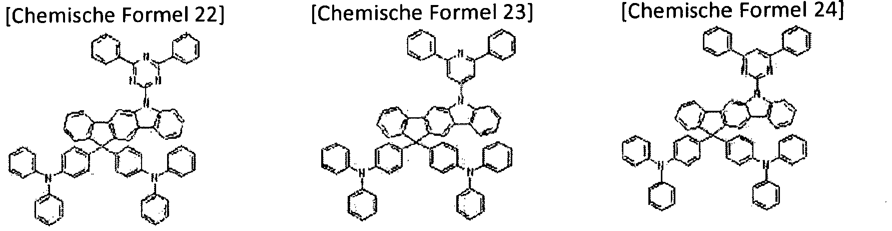

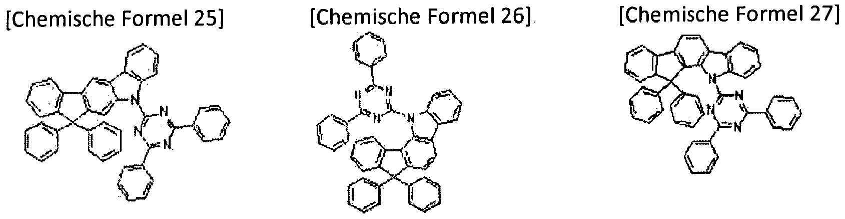

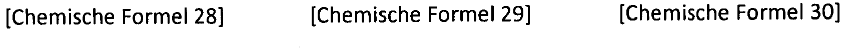

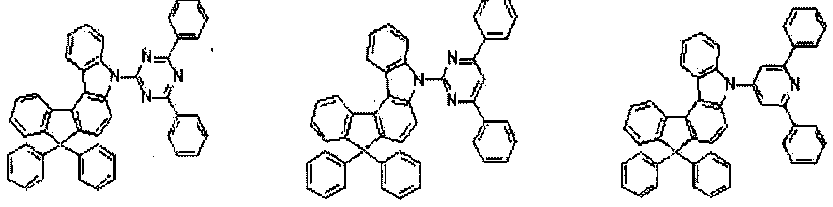

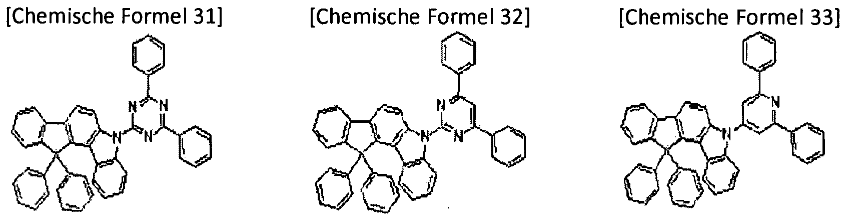

10. Die Verbindung für eine organisch photoelektrische Vorrichtung nach einem der Ansprüche 1 bis 9, wobei die Verbindung für eine organisch photoelektrische Vorrichtung eine thermische Zersetzungstemperatur (Td) von 350°C bis zu 600°C aufweist.

11. Verwendung einer Verbindung nach einem der Ansprüche 1 bis 10 als ein Ladungs-Transportmaterial oder als Host-Material in organisch photoelektrischen Vorrichtungen.

12. Eine organisch photoelektrische Vorrichtung umfassend
Eine Anode, eine Kathode, und wenigstens eine organische dünne Schicht eingefügt zwischen der Anode und der Kathode,
wobei wenigstens eine organisch dünne Schicht die Verbindung für eine organisch photoelektrische Vorrichtung nach einem der Ansprüche 1 bis 10 aufweist.

13. Die organisch photoelektrische Vorrichtung nach Anspruch 12, wobei die organische dünne Schicht eine Emissionsschicht, eine Löcher-blockierende Schicht, eine Elektronenblockierende Schicht, eine Elektronen-Transportschicht (ETL), eine Elektronen-injizierende Schicht (EIL), eine Löcher-injizierende Schicht (HIL), eine Löcher-Transportschicht (HTL), oder eine Kombination daraus ist.

14. Eine Bildschirm-Vorrichtung umfassend die organisch photoelektrische Vorrichtung nach Anspruch 12.

## Revendications

1. Composé pour un dispositif photoélectrique organique, dans lequel des substituants représentés par les Formules Chimiques 1 à 3 suivantes sont séquentiellement combinés : 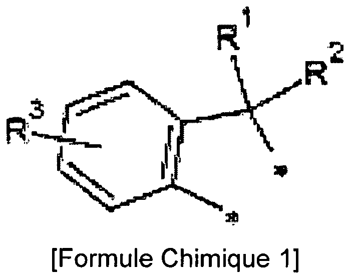 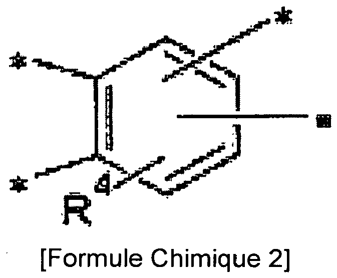 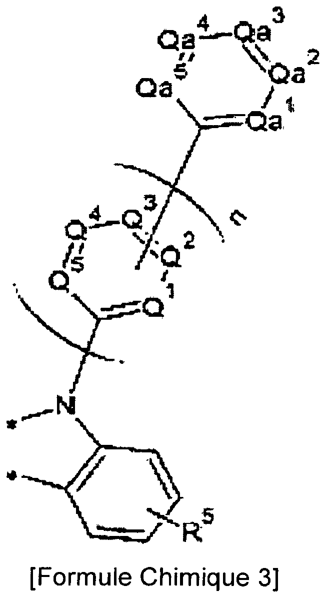 dans lequel, dans les Formules Chimiques 1 à 3,
R¹ et R² sont identiques ou différents et sont chacun indépendamment hydrogène, un groupe carbazolyle substitué ou non substitué, un groupe aryle en C6 à C30 substitué ou non substitué, un groupe hétéroaryle en C3 à C30 substitué ou non substitué, un groupe arylamine en C6 à C30 substitué ou non substitué, ou une combinaison de ceux-ci, R³ à R⁵ sont identiques ou différents et sont chacun indépendamment hydrogène, un groupe alkyle en C1 à C30, un groupe carbazolyle substitué ou non substitué, un groupe aryle en C6 à C30 substitué ou non substitué, un groupe hétéroaryle en C3 à C30 substitué ou non substitué, un groupe arylamine en C6 à C30 substitué ou non substitué, ou une combinaison de ceux-ci et
n est un nombre entier allant de 0 à 5,
et dans lequel dans la Formule Chimique 3 Q¹ à Q⁵ et Qa¹ à Qa⁵ sont identiques ou différents et sont chacun indépendamment N ou CR,
avec la condition que l'un ou plusieurs de Qa¹, Qa³ et Qa⁵ est N,
le reste de ceux-ci sont chacun indépendamment CR, et dans lequel le R est hydrogène, un groupe aryle en C6 à C30 substitué ou non substitué, un groupe hétéroaryle en C3 à C30 substitué ou non substitué, ou une combinaison de ceux-ci.

2. Composé pour un dispositif photoélectrique organique selon la revendication 1, dans lequel la Formule Chimique 1 est représentée par la Formule Chimique 1 a suivante : 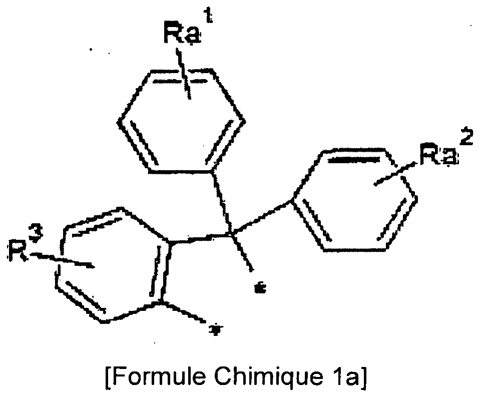 dans lequel, dans la Formule Chimique 1 a,
Ra¹ et Ra² sont identiques ou différents et sont chacun indépendamment hydrogène, un groupe carbazolyle substitué ou non substitué, un groupe aryle en C6 à C30 substitué ou non substitué, un groupe arylamine en C6 à C30 substitué ou non substitué, ou une combinaison de ceux-ci, et
R³ est hydrogène, un groupe carbazolyle substitué ou non substitué, un groupe aryle en C6 à C30 substitué ou non substitué, un groupe hétéroaryle en C3 à C30 substitué ou non substitué, un groupe arylamine en C6 à C30 substitué ou non substitué, ou une combinaison de ceux-ci.

3. Composé pour un dispositif photoélectrique organique selon la revendication 1 ou la revendication 2, dans lequel la Formule Chimique 2 est représentée par la Formule Chimique 2a ou la Formule Chimique 2b suivantes : 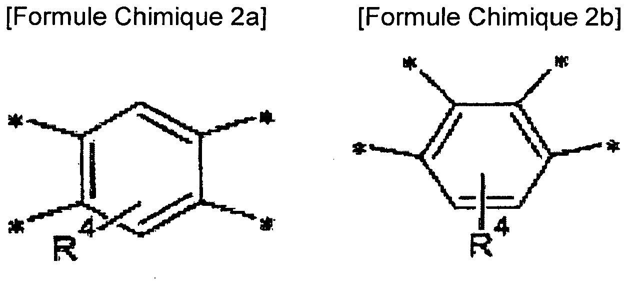 dans lequel, dans les Formules Chimiques 2a et 2b,
R⁴ est hydrogène, un groupe carbazolyle substitué ou non substitué, un groupe aryle en C6 à C30 substitué ou non substitué, un groupe hétéroaryle en C3 à C30 substitué ou non substitué, un groupe arylamine en C6 à C30 substitué ou non substitué, ou une combinaison de ceux-ci.

4. Composé pour un dispositif photoélectrique organique selon l'une quelconque des revendications 1 à 3, dans lequel, Q¹ à Q⁵ et Qa¹ à Qa⁵ dans la Formule Chimique 3 sont identiques ou différents et sont chacun indépendamment N ou CR,
avec la condition que un à trois choisi de Q¹ à Q⁵ est N,
un à trois choisi de Qa¹ à Qa⁵ est N, et
le reste de ceux-ci sont chacun indépendamment CR, dans lequel le R est hydrogène, un groupe aryle en C6 à C30 substitué ou non substitué, un groupe hétéroaryle en C3 à C30 substitué ou non substitué, ou une combinaison de ceux-ci.

5. Composé pour un dispositif photoélectrique organique selon l'une quelconque des revendications 1 à 4, dans lequel n dans la Formule Chimique 3 est 0 à 2.

6. Composé pour un dispositif photoélectrique organique selon l'une quelconque des revendications 1 à 5, dans lequel la Formule Chimique 3 est représentée par la Formule Chimique 3a suivante : 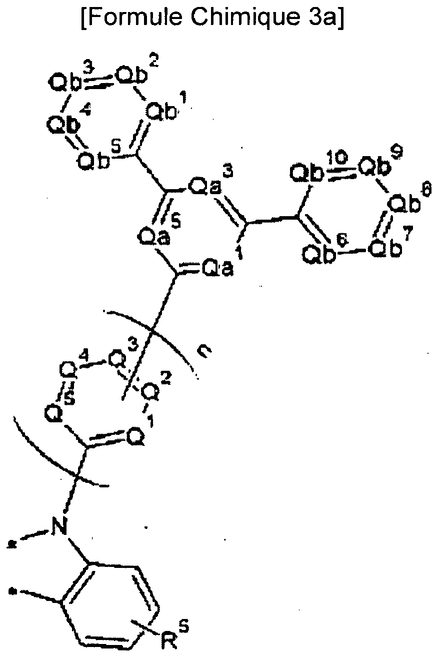 dans lequel, dans la Formule Chimique 3a,
Qa¹, Qa³ et Qa⁵ sont identiques ou différents, et sont chacun indépendamment N ou CH, avec la condition que l'un ou plusieurs de Qa¹, Qa³ et Qa⁵ est N,
Q¹ à Q⁵ et Qb¹ à Qb¹⁰ sont identiques ou différents et sont chacun indépendamment N ou CR, dans lequel R est hydrogène, un groupe aryle en C6 à C30 substitué ou non substitué, un groupe hétéroaryle en C3 à C30 substitué ou non substitué, ou une combinaison de ceux-ci,
R⁵ est hydrogène, un groupe carbazolyle, un groupe aryle en C6 à C30 substitué ou non substitué, un groupe hétéroaryle en C3 à C30 substitué ou non substitué, un groupe arylamine en C6 à C30 substitué ou non substitué, ou une combinaison de ceux-ci, et
n est un nombre entier allant de 0 à 5.

7. Composé pour un dispositif photoélectrique organique selon l'une quelconque des revendications 1 à 6, dans lequel dans les Formules Chimiques 1 à 3, R³ à R⁵ sont identiques ou différents et sont chacun indépendamment hydrogène, un groupe alkyle en C1 à C30, un groupe aryle en C6 à C30, ou une combinaison de ceux-ci.

8. Composé pour un dispositif photoélectrique organique selon l'une quelconque des revendications 1 à 7, dans lequel le composé pour le dispositif photoélectrique organique est représenté par les Formules Chimiques 4 à 9 suivantes : 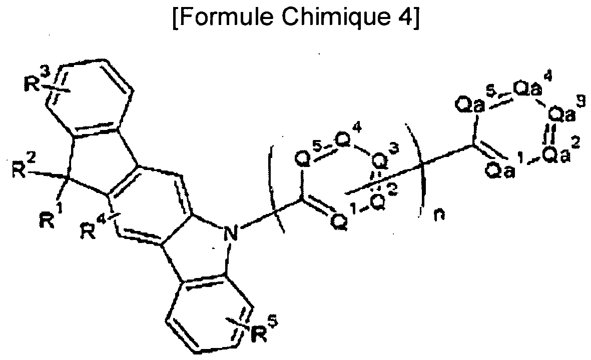 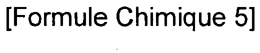 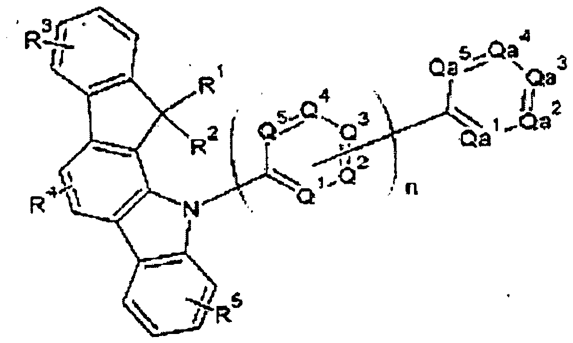 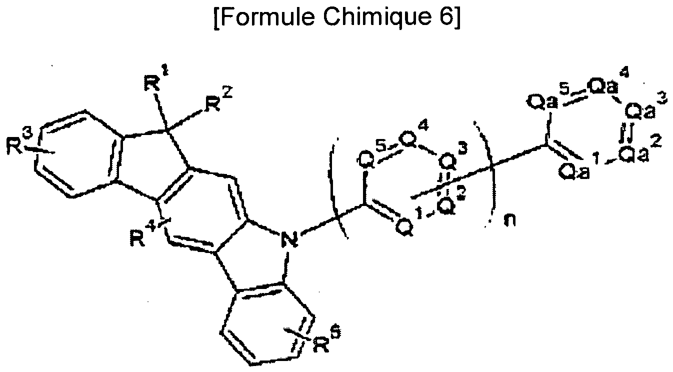 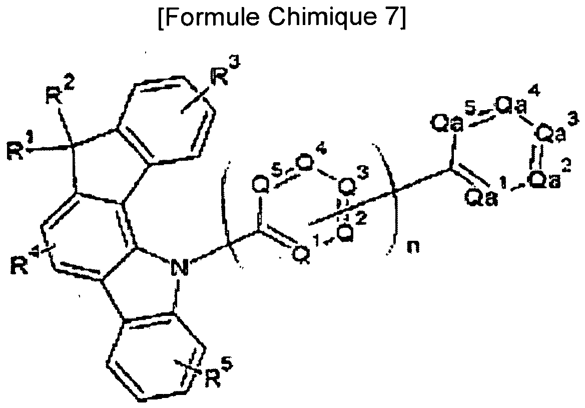 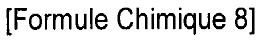 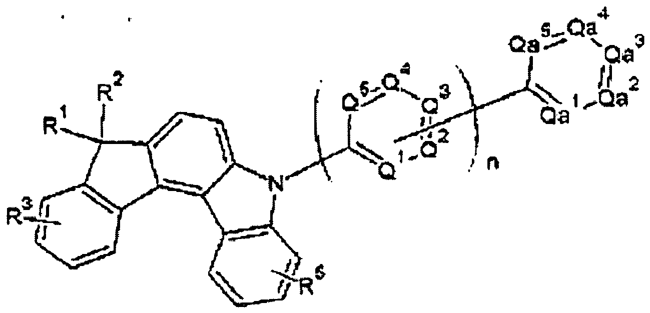 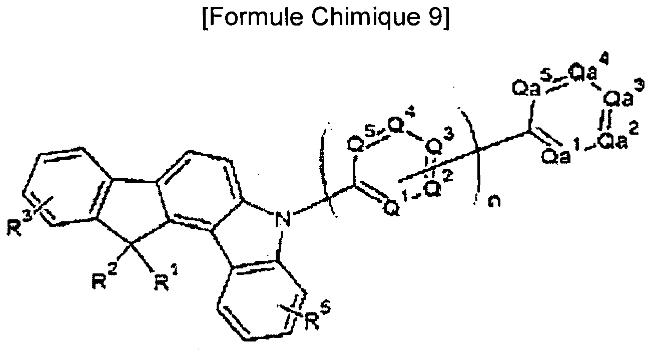 dans lequel, dans les Formules Chimiques 4 à 9,
Q¹ à Q⁵ et Qa¹ à Qa⁵ sont identiques ou différents et sont chacun indépendamment N ou CR, dans lequel R est hydrogène, un groupe aryle en C6 à C30 substitué ou non substitué, un groupe hétéroaryle en C3 à C30 substitué ou non substitué, ou une combinaison de ceux-ci,
R¹ à R⁵ sont identiques ou différents et sont chacun indépendamment hydrogène, un groupe carbazolyle substitué ou non substitué, un groupe aryle en C6 à C30 substitué ou non substitué, un groupe hétéroaryle en C3 à C30 substitué ou non substitué, un groupe arylamine en C6 à C30 substitué ou non substitué, ou une combinaison de ceux-ci, et
n est un nombre entier allant de 0 à 5.

9. Composé pour un dispositif photoélectrique organique selon l'une quelconque des revendications 1 à 8, dans lequel le composé pour le dispositif photoélectrique organique est un représenté par les Formules Chimiques 10 à 33 suivantes :

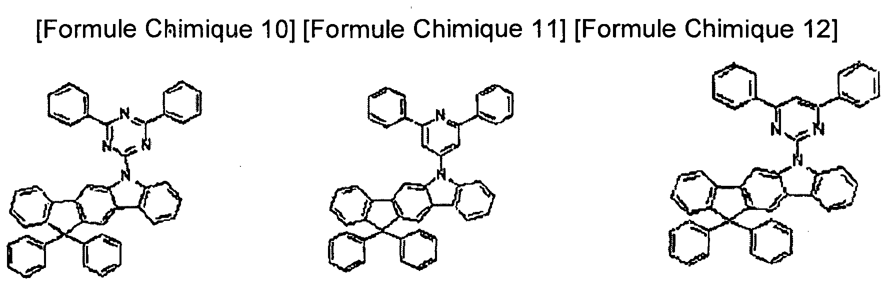

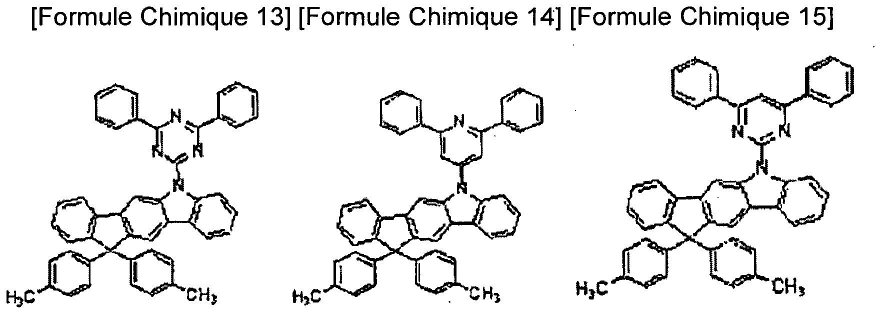

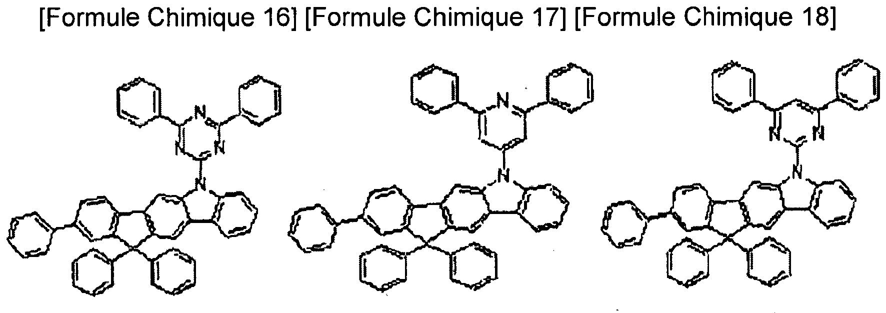

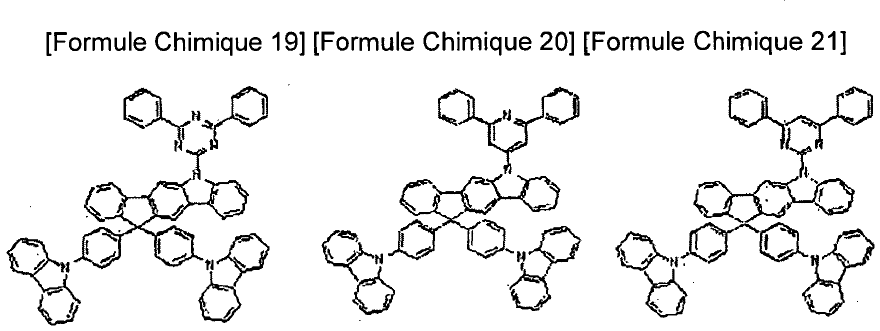

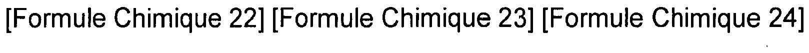

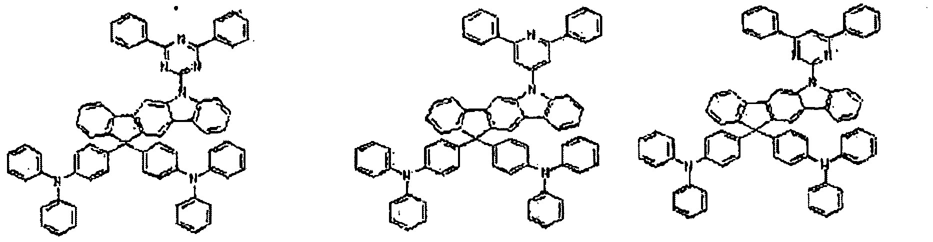

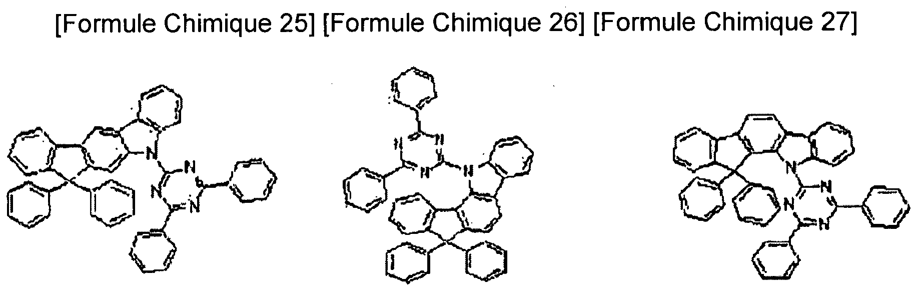

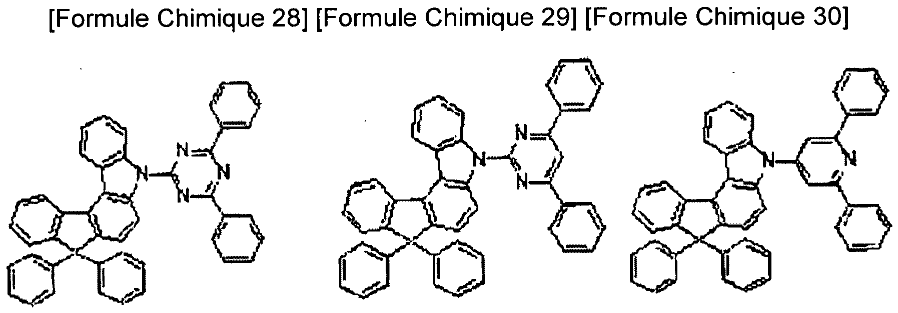

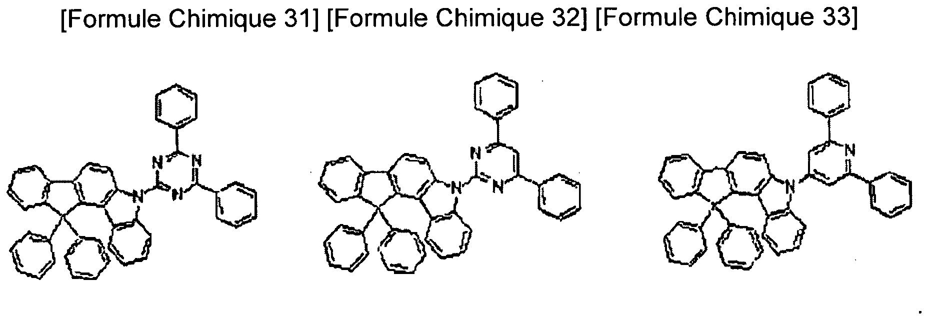

10. Composé pour un dispositif photoélectrique organique selon l'une quelconque des revendications 1 à 9, dans lequel le composé pour le dispositif photoélectrique organique a une température de décomposition thermique (Td) de 350°C à 600°C.

11. Utilisation d'un composé selon l'une quelconque des revendications 1 à 10 comme matériau de transport de charges ou matériau hôte dans des dispositifs photoélectriques organiques.

12. Dispositif photoélectrique organique comprenant
une anode, une cathode, et au moins une couche mince organique intercalée entre l'anode et la cathode,
dans lequel au moins une couche mince organique comprend le composé pour un dispositif photoélectrique organique selon l'une quelconque des revendications 1 à 10.

13. Dispositif photoélectrique organique selon la revendication 12, dans lequel la couche mince organique est une couche d'émission, une couche de blocage de trous, une couche de blocage d'électrons, une couche de transport d'électrons (ETL), une couche d'injection d'électrons (EIL), une couche d'injection de trous (HIL), une couche de transport de trous (HTL), ou une combinaison de celles-ci.

14. Dispositif d'affichage comprenant le dispositif photoélectrique organique selon la revendication 12.
